# EUROPEAN PATENT SPECIFICATION

(11) **EP 1 513 840 B1**
(45) Date of publication and mention of the grant of the patent: **04.10.2006**
(21) Application number: 03756326.9
(22) Date of filing: 30.05.2003
(51) Int. Cl.: C07D 471/04, A61K 31/4375, A61P 3/10

(54) **COMPOUNDS AND COMPOSITIONS FOR THE TREATMENT OF DIABETES AND DIABETES-RELATED DISORDERS**
VERBINDUNGEN UND ZUSAMMENSETZUNGEN ZUR BEHANDLUNG VON DIABETES UND ERKRANKUNGEN, DIE MIT DIABETES IN ZUSAMMENHANG STEHEN
COMPOSES ET COMPOSITIONS DE TRAITEMENT DU DIABETE ET DES TROUBLES ASSOCIES AU DIABETE

(30) Priority: 31.05.2002 US 384595 P
(43) Date of publication of application: 16.03.2005
(73) Proprietor: Bayer Pharmaceuticals Corporation, West Haven, CT 06516 (US)
(72) Inventor: WANG, Yamin, Sandy Hook, CT 06482 (US); MULL, Eric, S., Guilford, CT 06437 (US)
(74) Representative: Thomaier, Jörg
(86) International application number: PCT/US2003/017193
(87) International publication number: WO 2003/101988

(56) References cited:
- EP-A- 0 695 750
- WO-A-98/13350
- GB-A- 1 022 214

## Description

The present invention relates to novel compounds which are useful in the treatment of diabetes and diabetes-related disorders. The invention also relates to pharmaceutical compositions comprising said compounds, intermediates useful in the preparation of said compounds, and methods of preparation.

### BACKGROUND OF THE INVENTION

Diabetes is characterized by impaired glucose metabolism manifesting itself among other things by an elevated blood glucose level in the diabetic patient. Underlying defects lead to a classification of diabetes into two major groups: Type 1 diabetes, or insulin dependent diabetes mellitus (IDDM), arises when patients lack insulin-producing β-cells in their pancreatic glands. Type 2 diabetes, or non-insulin dependent diabetes mellitus (NIDDM), occurs in patients with impaired β-cell function and alterations in insulin action.

The current treatment for Type 1 diabetic patients is the administration of insulin by injection, while the majority of Type 2 diabetic patients are treated with agents that stimulate β-cell function or with agents that enhance the tissue sensitivity of the patients towards insulin. The drugs presently used to treat Type 2 diabetes include, for example, alpha-glucosidase inhibitors, insulin sensitizers, insulin secretagogues, and metformin.

Over time almost one-half of Type 2 diabetic subjects lose their response to these agents. Insulin treatment is instituted after diet, exercise, and oral medications have failed to adequately control blood glucose. The drawbacks of insulin treatment are the need for drug injection, the potential for hypoglycemia, and weight gain.

Because of the problems with current treatments, new therapies to treat Type 2 diabetes are needed. In particular, new treatments to maintain normal (glucose-dependent) insulin secretion are needed. Such new drugs should have the following characteristics: dependency on glucose for promoting insulin secretion (i.e., compounds that stimulate insulin secretion only in the presence of elevated blood glucose); low primary and secondary failure rates; and preservation of islet cell function. The strategy to develop the new therapy disclosed herein is based on the cyclic adenosine monophosphate (cAMP) signaling mechanism and its effects on insulin secretion.

Metabolism of glucose promotes the closure of ATP-dependent K+ channels, which leads to cell depolarization and subsequent opening of Ca++ channels. This in turn results in the exocytosis of insulin granules. cAMP is a major regulator of glucose-stimulated insulin secretion. However, it has little if any effect on insulin secretion in the absence of or at low glucose concentrations (Weinhaus, et al., Diabetes 47:1426-1435, 1998). The effects of cAMP on insulin secretion are thought to be mediated by a protein kinase A pathway.

Endogenous secretagogues like pituitary adenylate cyclase activating peptide (PACAP), VIP (vasoactive intestinal peptide), and GLP-1 (glucagon-like peptide-1) regulate insulin secretion in a glucose-dependent fashion via the cAMP system (Komatsu, et al., Diabetes 46:1928-1938, 1997). Also, phosphodiesterases (PDEs) are known to be involved in the regulation of the cAMP system.

PACAP is a potent stimulator of glucose-dependent insulin secretion from pancreatic β-cells. Three different PACAP receptor types (R1, R2, and R3) have been described (Harmar, et al., Pharmacol. Reviews 50:265-270, 1998). The insulinotropic action of PACAP is mediated by the GTP binding protein, Gs. Accumulation of intracellular cAMP in turn activates nonselective cation channels in β-cells increasing [Ca++]i, and promoting the exocytosis of insulin-containing secretory granules.

Vasoactive intestinal peptide (VIP) is a 28 amino acid peptide that was first isolated from hog upper small intestine (Said and Mutt, Science 169:1217-1218, 1970; U.S. Patent No. 3,879,371). This peptide belongs to a family of structurally related, small polypeptides that includes helodermin, secretin, the somatostatins, and glucagon. The biological effects of VIP are mediated by the activation of membrane-bound receptor proteins that are coupled to the intracellular cAMP signaling system. These receptors were originally known as V1P-R1 and VIP-R2, however, they were later found to be the same receptors as PACAP-R2 and PACAP-R3.

GLP-1 is released from intestinal L-cells after a meal and functions as an incretin hormone (i.e., it potentiates glucose-induced insulin release from the pancreatic β-cell). It is a 37-amino acid peptide that is differentially expressed by the glucagon gene, depending upon tissue type. The clinical data that support the beneficial effect of raising cAMP levels in β-cells have been established with GLP-1. Infusions of GLP-1 in poorly controlled Type 2 diabetics normalized their fasting blood glucose levels (Gutniak, et al., New Eng. J. Med. 326:1316-1322, 1992), and with longer infusions improved β-cell function as compared to those of normal subjects (Rachman, et al., Diabetes 45:1524-1530, 1996). A recent report has shown that GLP-1 improves the ability of β-cells to respond to glucose in subjects with impaired glucose tolerance (Byrne, et al., Diabetes 47:1259-1265, 1998). All of these effects, however, are short-lived because of the short half-life of the peptide.

### SUMMARY OF THE INVENTION

In one embodiment, the invention provides a compound of Formula (I) 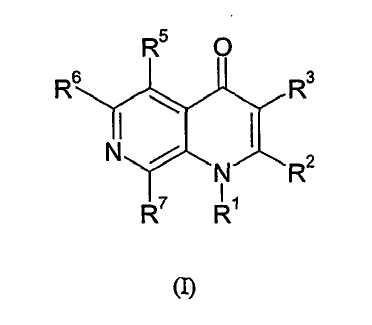 wherein

- R¹: is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can be substituted with up to 3 substituents selected from the group consisting of nitro, nitrile, halogen, alkyl of 1-4 carbon atoms, and alkoxy of 1-4 carbon atoms,
or
- R¹: is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms;
- R²: is -NR²⁻¹R²⁻² or -SR²⁻³;
R²⁻¹ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
R²⁻¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4. carbon atoms, and alkoxy of 1-4 carbon atoms;
R²⁻² is selected from the group consisting of hydrogen and alkyl of 1-6 carbon atoms,
or
R²⁻¹ and R²⁻² together with the nitrogen atom to which they are attached, form a heterocycloalkyl of 3-5 carbon atoms and 1-2 heteroatoms selected from N, O, and S, wherein said heterocycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻³ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
or
R²⁻³ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-3 carbon atoms;
- R³: is selected from the group consisting of hydrogen, halogen, and alkyl of 1-6 carbon atoms;
- R⁵: is selected from the group consisting of hydrogen, nitro, nitrile, halogen, hydroxy, amino, alkyl of 1-6 carbon atoms, haloalkyl of 1-6 carbon atoms, -OR⁵⁻¹ and -NR⁵⁻²R⁵⁻³,
or
- R⁵: is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻³ is selected from the group consisting of hydrogen and alkyl of 1-6 carbon atoms;
- R⁶: is selected from the group consisting of hydrogen, nitro, nitrile, halogen, hydroxy, amino, alkyl of 1-6 carbon atoms, haloalkyl of 1-6 carbon atoms, -OR⁶⁻¹, and NR⁶⁻²R⁶⁻³,
or
- R⁶: is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻³ is selected from the group consisting of hydrogen and alkyl of 1-6 carbon atoms;
- R⁷: is selected from the group consisting of hydrogen, nitro, nitrile, halogen, hydroxy, amino, alkyl of 1-6 carbon atoms, haloalkyl of 1-6 carbon atoms, -OR⁷⁻¹, and -NR⁷⁻²R⁷⁻³,
or
- R⁷: is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷⁻³ is selected from the group consisting of hydrogen and alkyl of 1-6 carbon atoms;
and a pharmaceutically acceptable salt thereof.

In another embodiment, the invention relates to a compound of Formula (I),
wherein
- R¹: is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, alkyl of 1-4 carbon atoms, haloalkyl of 1-4 carbon atoms, hydroxy and alkoxy of 1-4 carbon atoms;
- R²: is -NR²⁻¹R²⁻² ;
R²⁻¹ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
or
R²⁻¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻² is selected from the group consisting of hydrogen and alkyl of 1-6 carbon atoms,
or
R²⁻¹ and R²⁻² together with the nitrogen atom to which they are attached, form a heterocycloalkyl, wherein said heterocycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
- R³: is selected from the group consisting of hydrogen, halogen, and alkyl of 1-6 carbon atoms;
- R⁵: is selected from the group consisting of hydrogen, nitro, nitrile, halogen, hydroxy, amino, alkyl of 1-6 carbon atoms, haloalkyl of 1-6 carbon atoms, -OR⁵⁻¹, and -NR⁵⁻²R⁵⁻³,
or
- R⁵: is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, and cycloalkyl of 3-6 carbon atoms;
R⁵⁻² is selected from the group consisting of alkyl of 1-6 carbon atoms, haloalkyl of 1-6 carbon atoms, and cycloalkyl of 3-6 carbon atoms;
R⁵⁻³ is selected from the group consisting of hydrogen and alkyl of 1-6 carbon atoms;
- R⁶: is selected from the group consisting of hydrogen, nitro, nitrile, halogen, hydroxy, amino, alkyl of 1-6 carbon atoms, haloalkyl of 1-6 carbon atoms, and -OR⁶⁻¹;
R⁶⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
- R⁷: is selected from the group consisting of hydrogen, nitro, nitrile, halogen, hydroxy, amino, alkyl of 1-6 carbon atoms, haloalkyl of 1-6 carbon atoms, and -OR⁷⁻¹;
R⁷⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms; and a pharmaceutically acceptable salt thereof.

In another embodiment, the invention relates to a compound of Formula (I),
wherein
- R¹: is selected from the group consisting of phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said phenyl, thienyl, furyl, pyrrolyl, thiazolyl, oxazolyl, imidazolyl, pyridyl, pyrimidyl, pyridazinyl, cyclopropyl, cyclobutyl, cyclopentyl and cyclohexyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms;
- R²: is -NR²⁻¹R²⁻² ;
R²⁻¹ is selected from the group consisting of phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said phenyl, thienyl, furyl, pyrrolyl, thiazolyl, oxazolyl, imidazolyl, pyridyl, pyrimidyl, pyridazinyl, cyclopropyl, cyclobutyl, cyclopentyl and cyclohexyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻² is hydrogen;
- R³: is hydrogen;
- R⁵: is selected from the group consisting of hydrogen, nitro, nitrile, halogen, alkyl of 1-6 carbon atoms, haloalkyl of 1-6 carbon atoms, and -OR⁵⁻¹,
or
- R⁵: is selected from the group consisting of morpholino, piperazino, piperidino, pyrrolidino, phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said morpholino, piperazino, piperidino, pyrrolidino, phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, cyclopropyl, cyclobutyl, cyclopentyl, and cyclohexyl;
- R⁶: is selected from the group consisting of hydrogen, nitro, nitrile, halogen, alkyl of 1-6 carbon atoms, and haloalkyl of 1-6 carbon atoms;
- R⁷: is selected from the group consisting of hydrogen, nitro, nitrile, halogen, hydroxy, amino, alkyl of 1-6 carbon atoms, and haloalkyl of 1-6 carbon atoms;
and a pharmaceutically acceptable salt thereof.

In another embodiment, the invention relates to a compound of Formula (I),
wherein
- R¹: is phenyl, wherein said phenyl can optionally be substituted with up to 1 or 2 substituents selected from the group consisting of nitro, nitrile, fluoro, chloro, methyl, ethyl, propyl, butyl, trifluoromethyl, hydroxy, methoxy, and ethoxy;
- R²: is -NR²⁻¹R²⁻² ;
R²⁻¹ is phenyl wherein said phenyl can optionally be substituted with 1 or 2 substituents selected from the group consisting of nitro, nitrile, fluoro, chloro, hydroxy, methyl, ethyl, propyl, butyl, trifluoromethyl, methoxy, and ethoxy;
R²⁻² is hydrogen;
- R³: is hydrogen;
- R⁵: is selected from the group consisting of hydrogen, nitro, nitrile, halogen, methyl, ethyl, propyl, butyl, trifluoromethyl, and -OR⁵⁻¹,
or
- R⁵: is selected from the group consisting of phenyl, thienyl, pyridyl, pyrimidyl, cyclopropyl, cyclobutyl, cyclopentyl, and cyclohexyl, wherein said phenyl, thienyl, pyridyl, pyrimidyl, cyclopropyl, cyclobutyl, cyclopentyl, and cyclohexyl can optionally be substituted with 1 or 2 substituents selected from the group consisting of nitro, nitrile, fluoro, chloro, hydroxy, methyl, ethyl, propyl, butyl, trifluoromethyl, methoxy, and ethoxy;
R⁵⁻¹ is selected from the group consisting methyl, ethyl, propyl, butyl, trifluoromethyl, trifluoroethyl, cyclopropyl, cyclobutyl, cyclopentyl, and cyclohexyl;
- R⁶: is selected from hydrogen and methyl;
- R⁷: is selected from hydrogen and methyl;
and a pharmaceutically acceptable salt thereof.

In another embodiment, the invention relates to a medicament containing at least one such compound in combination with at least one pharmaceutically acceptable, pharmaceutically safe carrier or excipient; the use of such compounds for manufacturing a medicament for the treatment and/or prophylaxis of diabetes and diabetes-related disorders; such medicament for the treatment and/or prophylaxis of diabetes and diabetes-related disorders; and a process for controlling diabetes in humans and animals by administration of an insulinotropically effective amount of such compound.

### DETAILED DESCRIPTION OF THE INVENTION

### General Preparative Methods

The compounds of the invention may be prepared by use of known chemical reactions and procedures. Nevertheless, the following general synthetic schemes are presented to aid one skilled in the art in synthesizing compounds of this invention, with more detailed particular examples being presented below in the experimental section describing the working examples.

In general, compounds of Formula (I) may be prepared from the appropriately substituted nicotinic acid or nicotinic acid ester through several routes summarized in **Reaction Schemes I** to **III.** The starting nicotinic acid or nicotinic acid ester may be purchased commercially or prepared according to the literature in the field (*see, e.g.,* Chem. Pharm. Bull., 30:1257, 1982; Chem. Pharm. Bull., 24:2699, 1976; J. Med. Chem., 15:206, 1972; J. Amer. Chem. Soc., 119:1809, 1997; J. Chem. Soc., 18:2221-2226, 1996; U.S. Patent No. 5,962,481; U.S. Patent No. 5,571,775; U.S. Patent No. 5,614,469 and WO 00/64449).

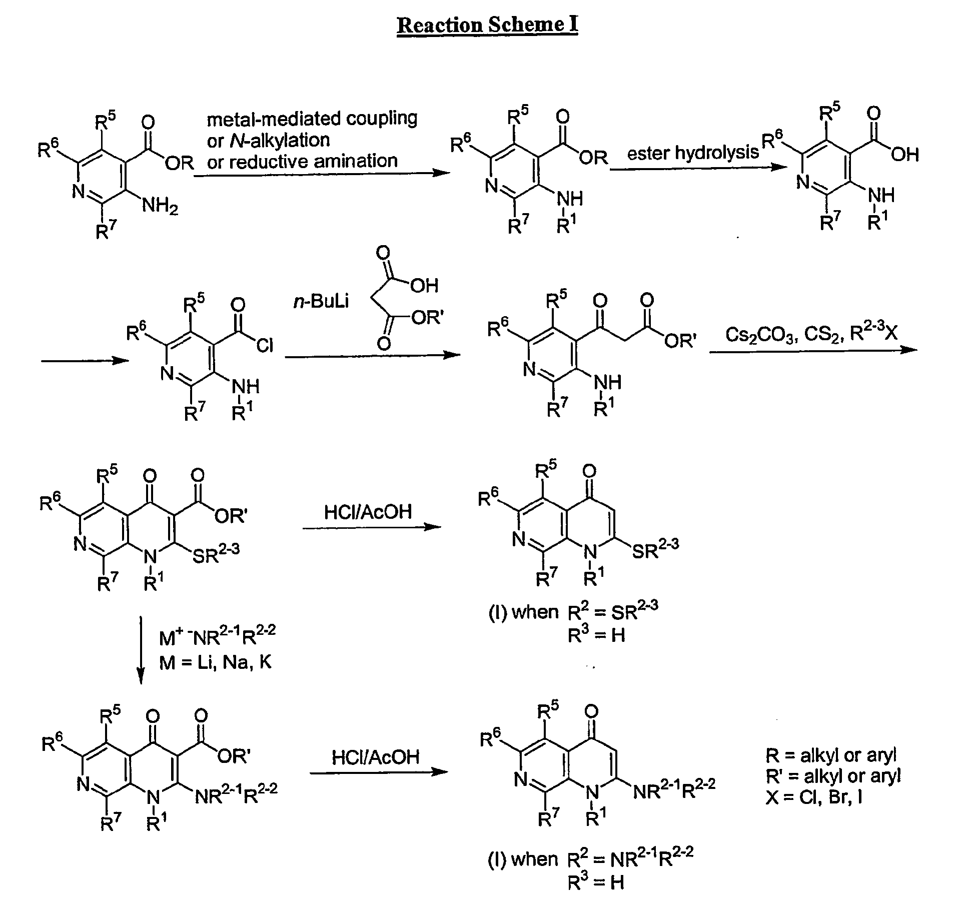

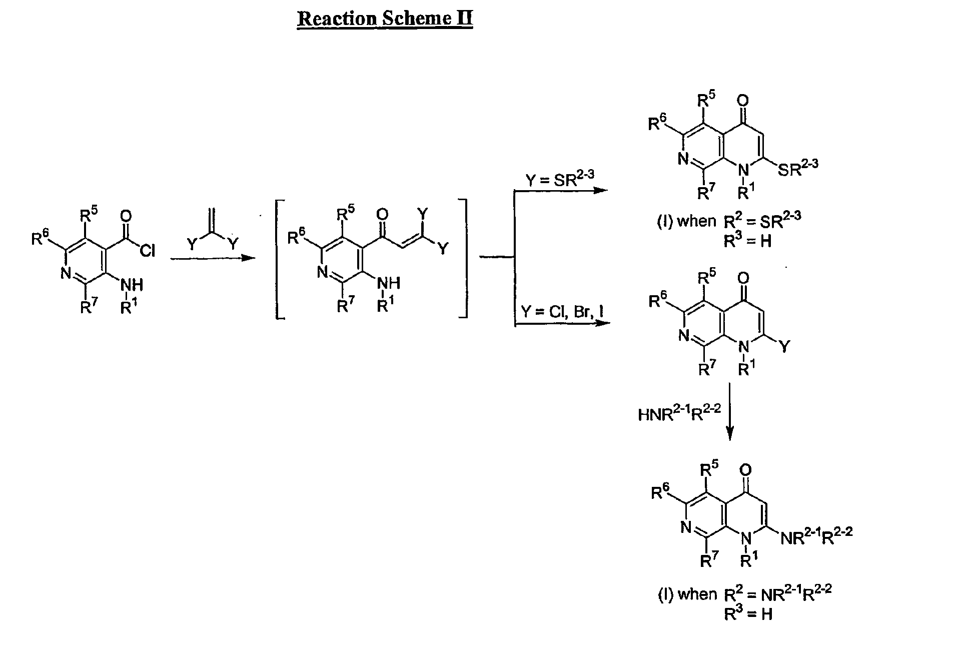

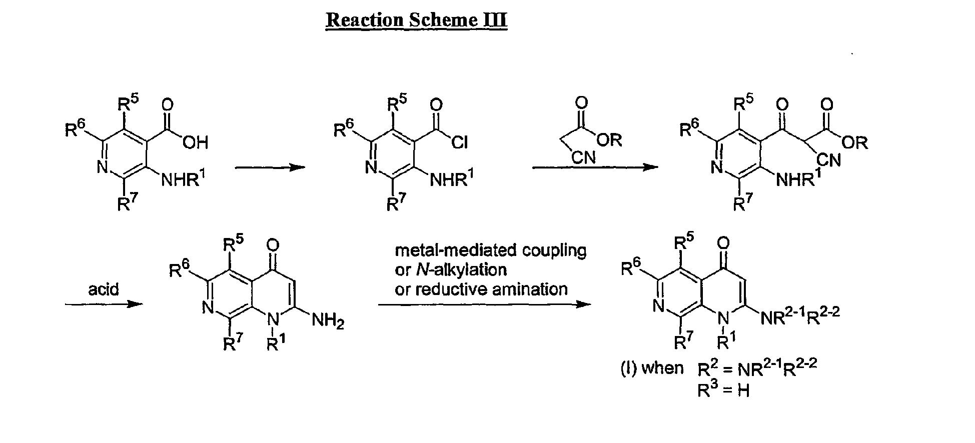

Further manipulations of compounds of Formula (I) could lead to more diversely substituted compounds. These manipulations include aromatic nucleophilic substitutions, metal-mediated couplings, alkylations, etc. **Reaction Scheme IV** illustrates transformations at R³ in Formula (I). **Reaction Scheme V** illustrates transformations at R⁵ in Formula (I). These transformations could also be applied to R⁶ and R⁷. **Reaction Scheme VI** illustrates transformations at R⁶ in Formula (I). These transformations could also be applied to R⁷.

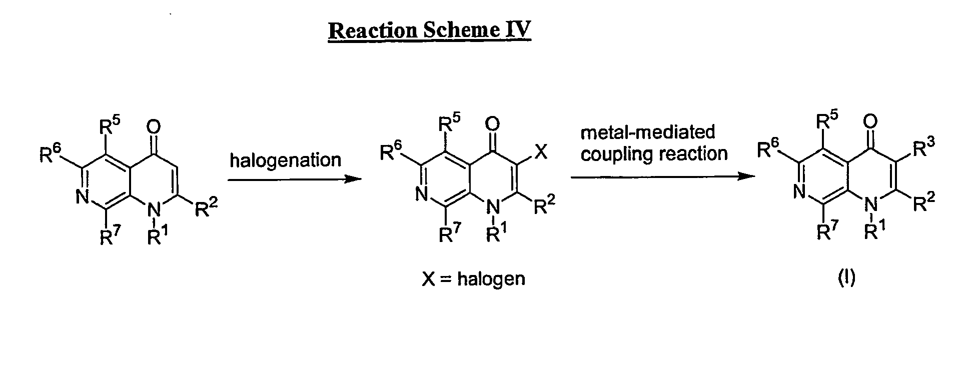

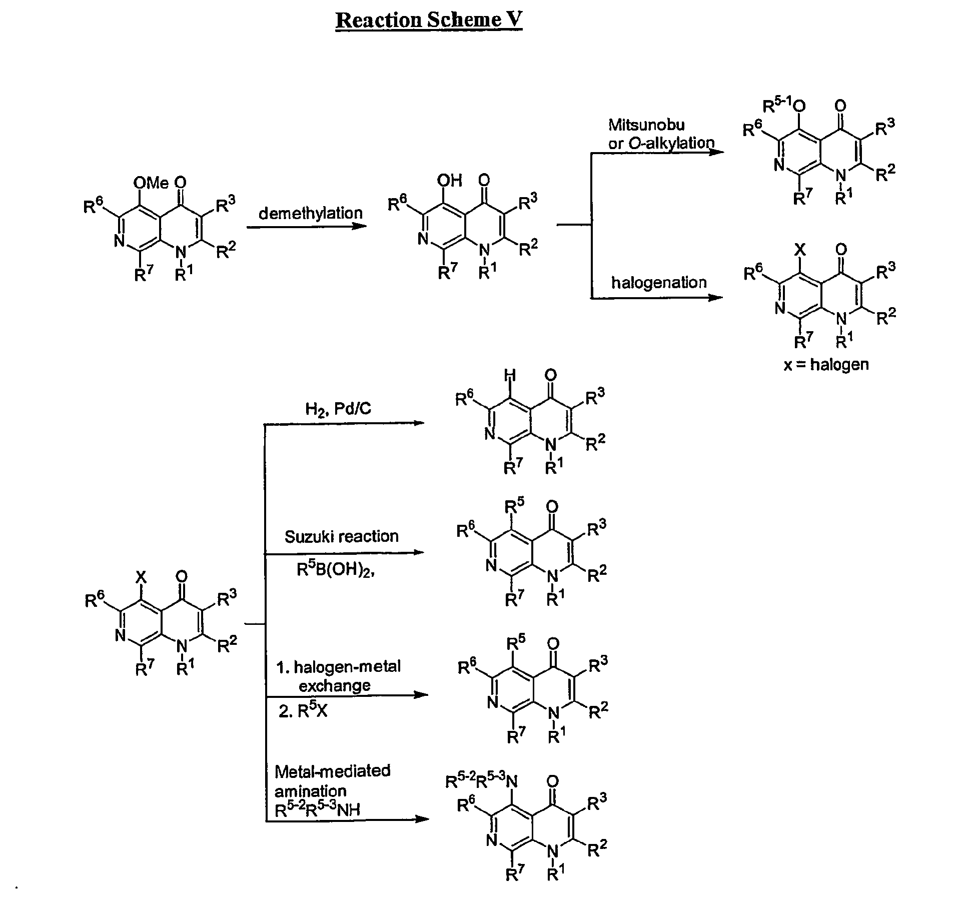

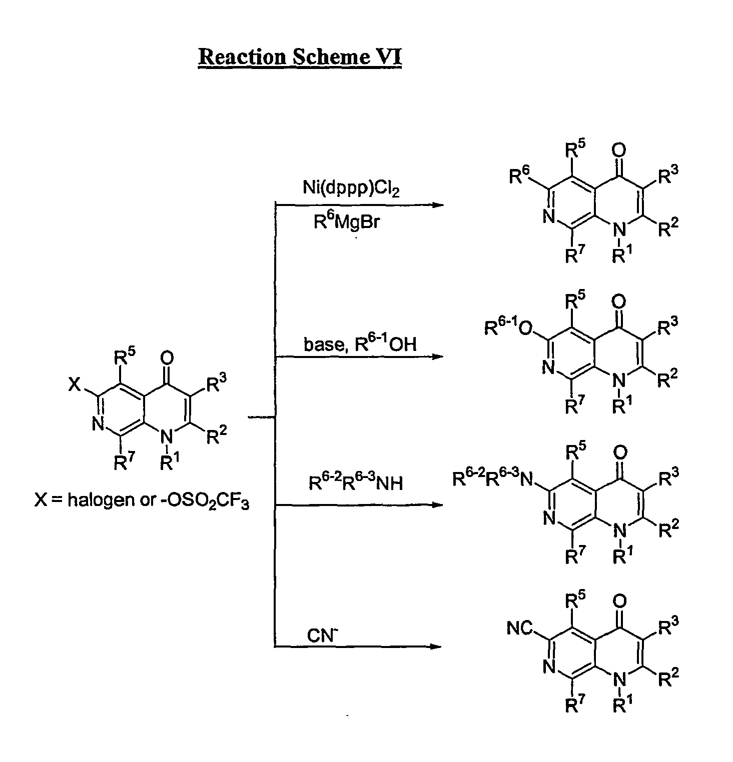

### Alternative Forms Of Novel Compounds

Also included in the compounds of the present invention are (a) the stereoisomers thereof, (b) the pharmaceutically-acceptable salts thereof, (c) the tautomers thereof, (d) the protected acids and the conjugate acids thereof, and (e) the prodrugs thereof.

### Stereoisomers

The stereoisomers of these compounds may include, but are not limited to, enantiomers, diastereomers, racemic mixtures and combinations thereof. Such stereoisomers can be prepared and separated using conventional techniques, either by reacting enantiomeric starting materials, or by separating isomers of compounds of the present invention. Isomers may include geometric isomers. Examples of geometric isomers include, but are not limited to, cis isomers or trans isomers across a double bond. Other isomers are contemplated among the compounds of the present invention. The isomers may be used either in pure form or in admixture with other isomers of the compounds described above.

### Pharmaceutically-Acceptable Salts

Pharmaceutically-acceptable salts of the compounds of the present invention include salts commonly used to form alkali metal salts or form addition salts of free acids or free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric, and phosphoric acid. Appropriate organic acids may be selected from aliphatic, cycloaliphatic, aromatic, heterocyclic, carboxylic, and sulfonic classes of organic acids. Examples of organic and sulfonic classes of organic acids includes, but are not limited to, formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicyclic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, N-hydroxybutyric, salicyclic, galactaric, and galacturonic acid and combinations thereof.

### Tautomers

Tautomers of the compounds of the invention are encompassed by the present invention. Thus, for example, a carbonyl includes its hydroxy tautomer.

### Protected Acids and the Conjugate Acids

The protected acids include, but are not limited to, protected acids such as esters, hydroxyamino derivatives, amides, and sulfonamides.

### Prodrugs

The present invention also includes the prodrugs and salts of the prodrugs. Formation of prodrugs is well known in the art in order to enhance the properties of the parent compound; such properties include solubility, absorption, biostability, and release time *(see, e.g., "Pharmaceutical Dosage Form and Drug Delivery Systems"* (Sixth Edition), ed. Ansel et al., Williams & Wilkins, pg. 27-29, 1995, which is hereby incorporated by reference). Commonly used prodrugs are designed to take advantage of the major drug biotransformation reactions and are also to be considered within the scope of the invention. Major drug biotransformation reactions include N-dealkylation, O-dealkylation, aliphatic hydroxylation, aromatic hydroxylation, N-oxidation, S-oxidation, deamination, hydrolysis reactions, glucuronidation, sulfation, and acetylation *(see, e.g., Goodman and Gilman's The Pharmacological Basis of Therapeutics* (Ninth Edition), ed. Molinoff et al., McGraw-Hill, pg 11-13, 1996, which is hereby incorporated by reference).

### Solvates

The present invention includes the solvates and the solvates of the salts. Solvates for the purposes of the invention are those forms of the compounds that coordinate with solvent molecules to form a complex in the solid or liquid state. Hydrates are a specific form of solvates, where the coordination is with water. These include, but are not limited to, monohydrates and semihydrates.

### Definitions

A comprehensive list of the abbreviations utilized by organic chemists of ordinary skill in the art appears in the first issue of each volume of the *Journal of Organic Chemistry;* this list is typically presented in a table entitled Standard List of Abbreviations. The abbreviations contained in said list, and all abbreviations utilized by organic chemists of ordinary skill in the art are hereby incorporated by reference.

For purposes of this invention, the chemical elements are identified in accordance with the Periodic Table of the Elements, CAS version, Handbook of Chemistry and Physics, 67th Ed., 1986-87.

For the purposes of the present invention, the subsituents have the following meanings, unless otherwise specified:

Alkyl represents a linear or branched alkyl radical having generally 1 to 8 ("of 1-8 carbon atoms"), preferably 1 to 4 carbon atoms. Examples of an "alkyl" group include methyl, ethyl, n-propyl, isopropyl, tert-butyl, n-pentyl, n-hexyl, and the like.

Haloalkyl represents a linear or branched alkyl substituted up to perhalo level with halogen, preferably with chlorine or fluorine. Examples of a "halolakyl" group include trifluoromethyl, and the like.

Alkoxy represents an alkyl group linked to a second group via an oxygen atom. Eamples of an "alkoxy" group include methoxy, ethoxy, n-propoxy, isopropoxy, tert-butoxy, and the like.

Cycloalkyl represents a monocyclic analog of an alkyl group having 3 to 8 carbon atoms as defined above. Examples of a "cycloalkyl" group include cyclopropyl, cyclobutyl, cyclopentyl, cyclohexyl, cycloheptyl, and the like.

Heteroaryl represents an aromatic monocyclic radical of 3-5 carbon atoms and 1-2 heteroatoms selected from N, O, and S. Examples of a "heteroaryl" group include thienyl, furyl, pyrrolyl, thiazolyl, oxazolyl, imidazolyl, pyridyl, pyrimidyl, pyridazinyl, and the like.

Heterocycloalkyl of 3-5 carbon atoms and 1-2 heteroatoms selected from N, O, and S represents a monocyclic cycloalkyl group having 4 to 7, preferably 5 or 6 atoms where one or more of the carbon atoms are replaced by one or more atoms independently chosen from nitrogen, oxygen, or sulfur atoms. Unless otherwise specified, the heterocycloalkyl ring may be attached at any carbon atom or heteroatom that results in a stable structure and, if substituted, may be substituted at any suitable carbon atom or heteroatom which results in a stable structure. Examples of a "heterocycloalkyl" group include morpholino, piperazino, piperidino, pyrrolidino, tetrahydrofurano, and the like.

Halogen represents fluorine, chlorine, bromine, and iodine.

The terms "optional" or "optionally" mean that the subsequently described event or circumstances may or may not occur, and that the description includes instances where the event or circumstance occurs and instances in which it does not. For example, "optionally substituted alkyl" means that the alkyl radical may or may not be substituted and that the description includes both substituted alkyl radicals and alkyl radicals having no substitution.

### Abbreviations and Acronyms

When the following abbreviations are used throughout the disclosure, they have the following meaning:
- CH₂Cl₂: methylene chloride
- THF: tetrahydrofuran
- DMSO: dimethylsulfoxide
- EtOAc: ethyl acetate
- Et₃N: triethylamine
- HCl: hydrochloric acid
- ¹H NMR: proton nuclear magnetic resonance
- HPLC: high performance liquid chromatography
- K₂CO₃: potassium carbonate
- NH₄Cl: ammonium chloride
- LC/MS: liquid chromatography / mass spectroscopy
- MeOH: methanol
- RT: retention time
- h: hour
- min: minutes
- DMF: *N,N*-dimethylformamide
- BuLi: butyllithium
- TLC: thin layer chromatography
- TFA: trifluoacetic acid
- LiHMDS: lithium hexamethyldisilazide
- LDA: lithium diisopropylamide
- AcOH: acetic acid

### EXPERIMENTAL PROCEDURES

All reactions were carried out under a positive pressure of dry argon or dry nitrogen, and were stirred magnetically unless otherwise indicated. Sensitive liquids and solutions were transferred via syringe, and introduced into reaction vessels through rubber septa. Commercial grade reagents and solvents were used without further purification.

Unless otherwise stated, the term "concentration under reduced pressure" refers to use of a Buchi rotary evaporator at approximately 15 mm Hg. All temperatures are reported uncorrected in degrees Celsius (°C). Unless otherwise indicated, all parts and percentages are by volume.

Proton (¹H) nuclear magnetic resonance (NMR) spectra were measured with a Varian Mercury (300 MHz) or a Bruker Avance (500 MHz) spectrometer with either Me₄Si (δ 0.00) or residual protonated solvent (CHCl₃ δ 7.26; MeOH δ 3.30; DMSO δ 2.49) as standard. The NMR data of the synthesized examples, some of which are not disclosed in the following detailed examples, are in agreement with their corresponding structural assignments.

The HPLC-MS spectra were obtained using a Hewlett-Packard 1100 HPLC equipped with a quaternary pump, a variable wavelength detector set at 254 nm, a YMC pro C-18 column (2 x 23 mm, 120A), and a Finnigan LCQ ion trap mass spectrometer with electrospray ionization. Spectra were scanned from 120-1200 amu using a variable ion time according to the number of ions in the source. The eluents were A: 2% CH₃CN in water with 0.02% TFA and B: 2% water in CH₃CN with 0.018% TFA. Gradient elution from 10% to 95% B over 3.5 minutes at a flow rate of 1.0 mL/min was used with an initial hold of 0.5 minutes and a final hold at 95% B of 0.5 minutes. Total run time was 6.5 minutes.

The IUPAC Name was obtained using the ACD/ILab Web service.

The following specific Examples are presented to illustrate the invention described herein, but they should not be construed as limiting the scope of the invention in any way. It should be apparent to one of ordinary skill in the art that changes and modifications can be made to this invention without departing from the spirit or the scope of the invention.

### Intermediates and exemplary compounds:

### INTERMEDIATE 1

### Ethyl 3-(3-anilino-4-pyridinyl)-3-oxopropanoate

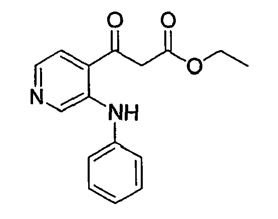

3-Anilinoisonicotinic acid (413 mg, 1.92 mmol) (prepared according to J. Org. Chem. 14:97-101, 1949) and one drop of DMF were suspended in CH₂Cl₂(30 mL). To the resulting mixture was added oxalyl chloride (0.84 mL, 9.65 mmol) slowly at 0°C. After 1 hour of stirring, the reaction mixture was concentrated under reduced pressure to give the acid chloride. To a separate flask containing ethyl hydrogen malonate (0.53 mL, 4.49 mmol) in THF (20 mL) at -40°C was added n-BuLi (2.5 M in Hex, 0.53 mL, 8.89 mmol). The solution became a white suspension and was stirred at -40°C for 2 h before cooling to -78°C. To the above mixture was added acid chloride in THF (20 mL) at -78°C. The reaction mixture was stirred at -78 °C for 30 minutes, warmed to room temperature, stirred for an additional 1 hour, and quenched with water. Solvents were removed under reduced pressure and the crude product was purified by a Biotage column and eluting with 15% EtOAc/Hex to give ethyl 3-(3-anilino-4-pyridinyl)-3-oxopropanoate as a yellow oil (80 mg, 15%). RT (HPLC): 2.34 min; MH⁺: 285.

### INTERMEDIATE 2

### Ethyl 2-(methylsulfanyl)-4-oxo-1-phenyl-1,2,3,4-tetrahydro-1,7-naphthyridine-3-carboxylate

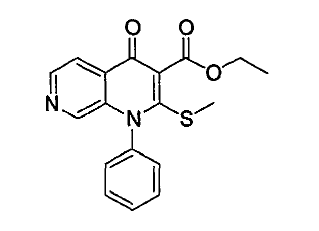

Ethyl 3-(3-anilino-4-pyridinyl)-3-oxopropanoate (80 mg, 0.28 mmol) was dissolved in THF (4 mL) and cooled to -10°C. To this solution was added Cs₂CO₃ (228 mg, 0.7 mmol). The reaction mixture was stirred for 15 minutes before CS₂ (0.044 mL, 1.4 mmol) was added. The reaction was stirred at -10°C for an additional 2 h at which time MeI (0.044 mL, 0.7 mmol) was added. After another 4 hours of stirring, more Cs₂CO₃ (91 mg, 0.21 mmol) was added to the reaction mixture and the reaction was stirred overnight. The crude reaction mixture was diluted with EtOAc, washed with water, and brine. The organic layer was dried over Na₂SO₄. The crude product was purified by preparative reverse-phase HPLC to give ethyl 2-(methylsulfanyl)-4-oxo-1-phenyl-1,2,3,4-tetrahydro-1,7-naphthyridine-3-carboxylate as a brown solid (30 mg, 28%): RT (HPLC): 2.45 min, MH⁺: 341.

### INTERMEDIATE 3

### Ethyl 2-anilino-4-oxo-1-phenyl-1,4-dihydro-1,7-naphthyridine-3-carboxylate

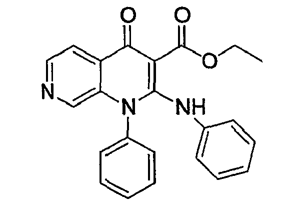

To a solution of aniline (22 mg, 0.18 mmol) in THF (2 mL) at-40°C was added LiHMDS. After 1 hour, a solution of ethyl 2-(methylsulfanyl)-4-oxo-1-phenyl-1,2,3,4-tetrahydro-1,7-naphthyridine-3-carboxylate (30 mg, 0.088 mmol) in THF (1 mL) was added dropwise. After 20 minutes, the reaction was quenched with brine and the mixture was extracted with CH₂Cl₂. The combined organic layers were washed with water, and dried over Na₂SO₄. The crude product was purified by preparative reverse-phase HPLC to give ethyl 2-anilino-4-oxo-1-phenyl-1,4-dihydro-1,7-naphthyridine-3-carboxylate as a yellow solid (15 mg, 44%). RT (HPLC): 2.24 min; MH⁺: 386.

### EXAMPLE 1

### 2-anilino-1-phenyl-1,7-naphthyridin-4(1H)-one

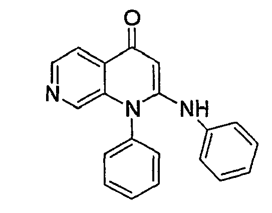

A 2:1 mixture of HC1/HOAc (3 mL) was added to ethyl 2-anilino-4-oxo-1-phenyl-1,4-dihydro-1,7-naphthyridine-3-carboxylate (13 mg, 0.034 mmol) and the reaction was heated to 120°C for 18 h. The mixture was cooled to room temperature, and neutralized with aqueous NaOH (20%), and was extracted with EtOAc. The combined organic layers were washed with saturated NaHCO₃, brine, and dried (Na₂SO₄). Solvents were removed under reduced pressure and the residue was trituated with Et₂O to provide 2-anilino-1-phenyl-1,7-naphthyridin-4(1H)-one (5 mg, 47%). RT (HPLC): 1.77 min; MH⁺: 314.

Utilizing the above described procedures for intermediates and Examples, and Reaction Schemes I - VI alone or in combination, a variety of Formula (I) compounds can be prepared using the appropriate starting material. Exemplary compounds are listed in Table 1.

**TABLE 1**

| **Example** | **Structure** |
|---|---|
| 2 | 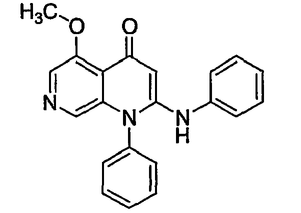 |
| 3 | 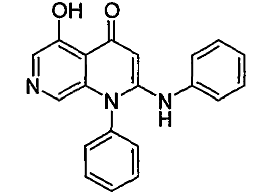 |
| 4 | 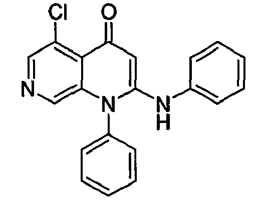 |
| 5 | 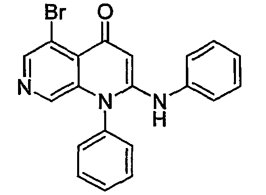 |
| 6 | 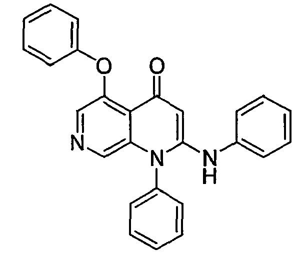 |
| 7 | 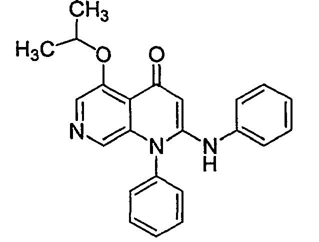 |
| 8 | 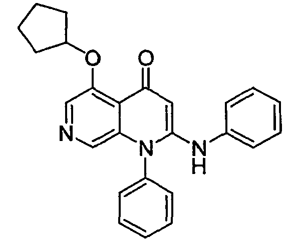 |
| 9 | 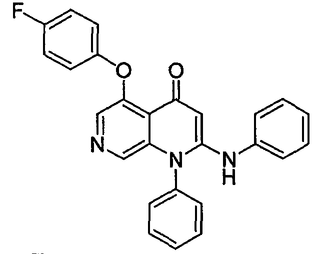 |
| 10 | 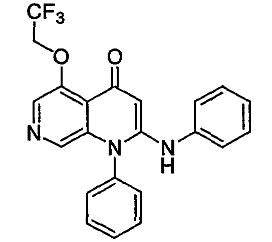 |
| 11 | 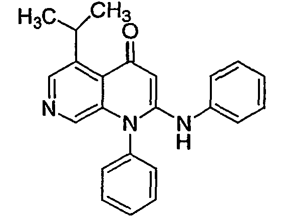 |
| 12 | 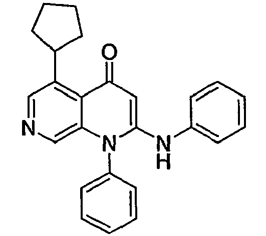 |
| 13 | 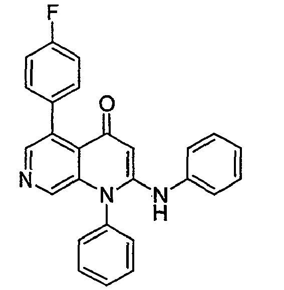 |
| 14 | 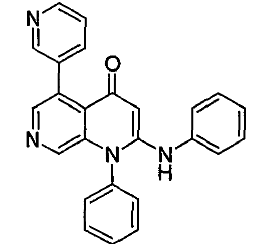 |
| 15 | 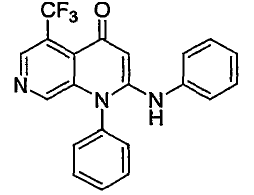 |
| 16 | 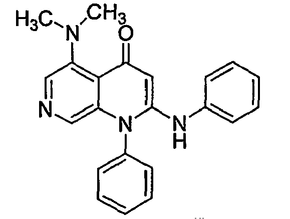 |
| 17 | 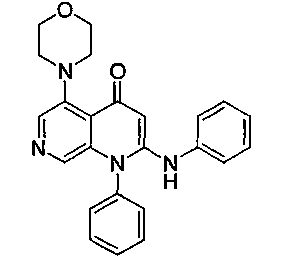 |
| 18 | 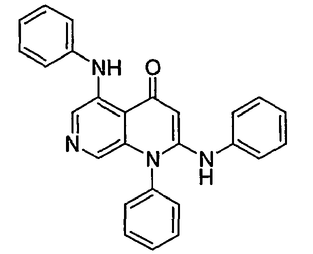 |
| 19 | 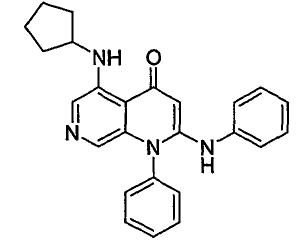 |
| 20 | 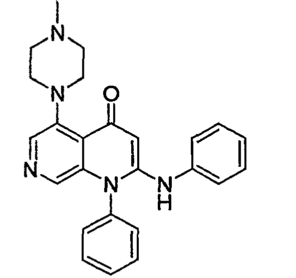 |
| 21 | 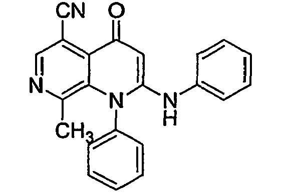 |
| 22 | 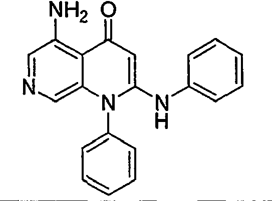 |
| 23 | 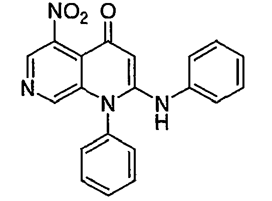 |
| 24 | 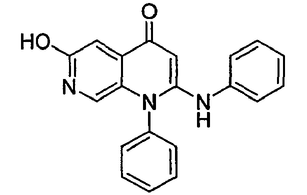 |
| 25 | 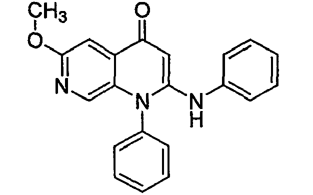 |
| 26 | 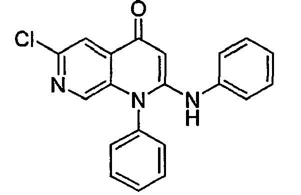 |
| 27 | 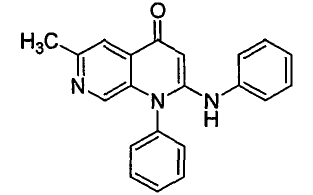 |
| 28 | 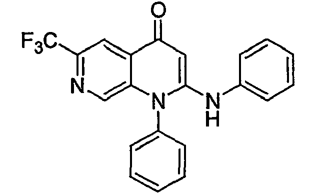 |
| 29 | 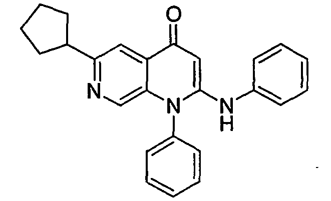 |
| 30 | 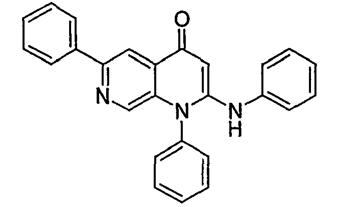 |
| 31 | 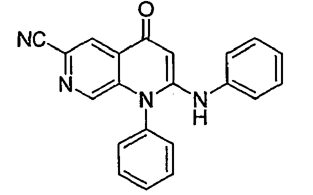 |
| 32 | 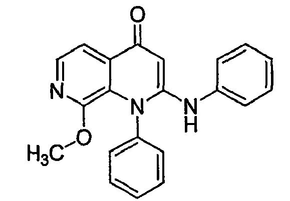 |
| 33 | 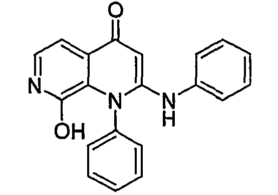 |
| 34 | 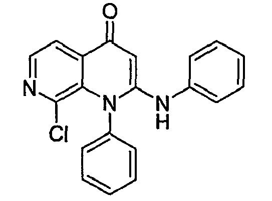 |
| 35 | 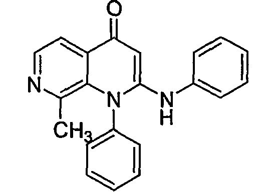 |
| 36 | 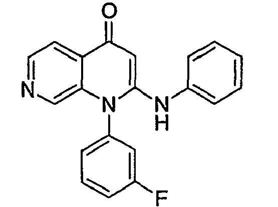 |
| 37 | 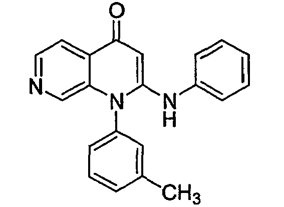 |
| 38 | 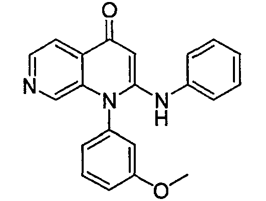 |
| 39 | 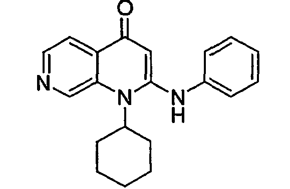 |
| 40 | 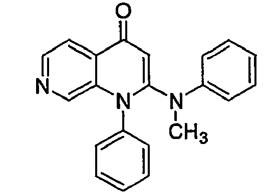 |
| 41 | 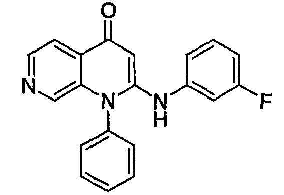 |
| 42 | 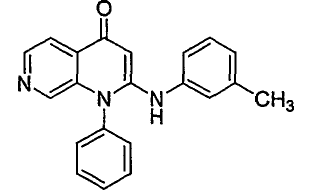 |
| 43 | 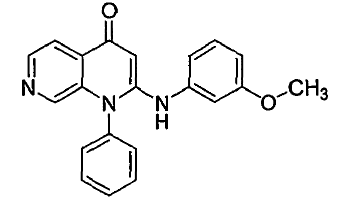 |
| 44 | 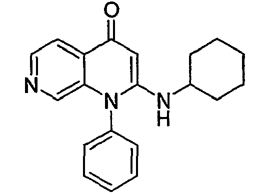 |
| 45 | 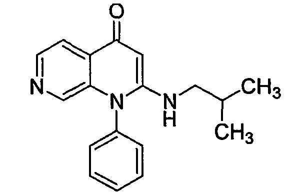 |
| 46 | 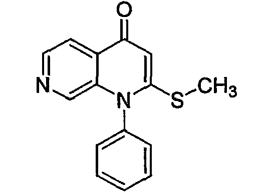 |
| 47 | 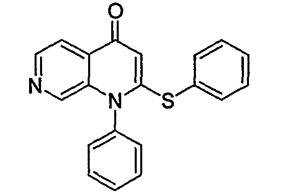 |
| 48 | 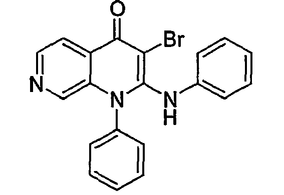 |
| 49 | 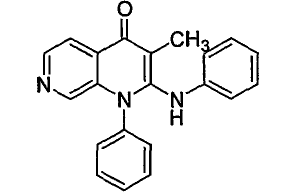 |

The present application also relates to a process for preparing a compound of the present invention, wherein a compound of Formula (II), 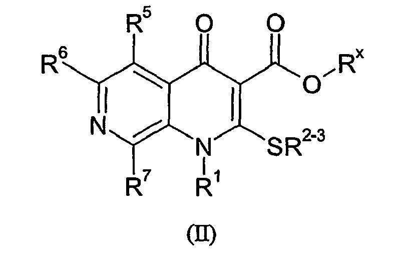 wherein R^{x} represents an alkyl group, such as methyl or ethyl, preferably ethyl, and R¹ to R⁷ are as defined above,
is decarboxylated, for example by reaction in acidic medium, such as hydrochloric acid in acetic acid.

A compound of Formula (II) can be prepared by first reacting a compound of Formula (III) 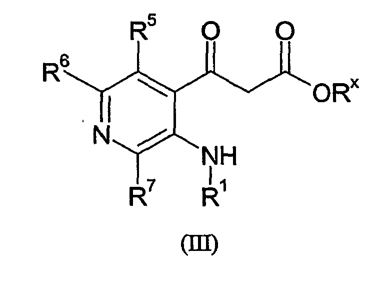 wherein R^{x} and R¹ to R⁷ are as defined above,
with carbon disulfide in the presence of a base, preferably cesium carbonate, followed by reacting the intermediate with a compound of Formula (IV)

X-R²⁻³ (IV),

wherein X represents a leaving group, such as a halogen, preferably iodine and bromine, and R²⁻³ is as defined above.

A compound of Formula (III) can be prepared by first converting a compound of Formula (V) 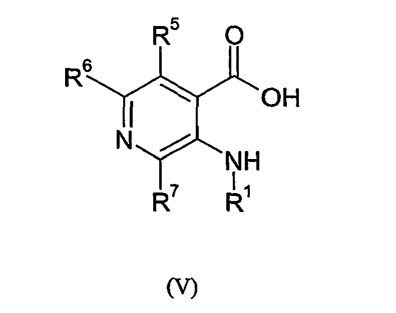 wherein R¹ and R⁵ to R⁷ are as defined above,
into the acid chloride, for example, by reaction with oxalyl chloride, followed by reaction with a compound of Formula (VI) 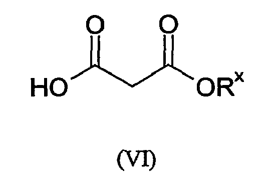 wherein R^{x} is as defined above, in the presence of a base, preferably *n*-BuLi.

Alternatively, 3-anilinoisonicotinic acid (prepared according to J. Org. Chem. 14:97-101, 1949) can be used instead of a compound of Formula (V), to yield the unsubstituted ring system, which is substituted with substituents R⁵, R⁶, and R⁷at a later stage.

A compound of Formula (I), wherein R² represents -NR²⁻¹R²⁻² , wherein R²⁻¹ and R²⁻² are as described above, can be prepared by reacting a compound of Formula (II) with a compound of Formula (VI)

HNR²⁻¹R²⁻² (VI),

wherein R²⁻¹ and R²⁻² are as described above,
followed by decarboxylation.

The compounds of Formulae (IV), (V), and (VI) are known from the literature or can be prepared according to procedures known from the literature. The nicotinic acids of Formula (V) could be prepared according to literature in this field (*see, e.g.,* Chem. Pharm. Bull., 30:1257, 1982; Chem. Pharm. Bull., 24:2699, 1976; J. Mcd. Chem., 15:206, 1972; J. Amer. Chem. Soc., 119:1809, 1997; J. Chem. Soc., 18:2221-2226, 1996; U.S. Patent No. 5,962,481; U.S. Patent No. 5,571,775; U.S. Patent No. 5,614,469 and WO 00/64449).

### Use

The present invention also includes the use of compounds of the invention for the treatment of diabetes and related diseases and conditions.

Compounds of the invention may be used to treat diseases, such as diabetes, including both Type 1 and Type 2 diabetes. Such methods may also delay the onset of diabetes and diabetic complications. Other diseases and conditions that may be treated or prevented using compounds of the invention include: Maturity-Onset Diabetes of the Young (MODY) (Herman, et al., Diabetes 43:40, 1994); Latent Autoimmune Diabetes Adult (LADA) (Zimmet, et al., Diabetes Med. 11:299, 1994); impaired glucose tolerance (IGT) (Expert Committee on Classification of Diabetes Mellitus, Diabetes Care 22 (Supp. 1):S5, 1999); impaired fasting glucose (IFG) (Charles, et al., Diabetes 40:796, 1991); gestational diabetes (Metzger, Diabetes, 40:197, 1991); and metabolic syndrome X.

In addition, the compounds of the present invention may also be effective in such disorders as obesity, and in the treatment of atherosclerotic disease, hyperlipidemia, hypercholesteremia, low HDL levels, hypertension, cardiovascular disease (including atherosclerosis, coronary heart disease, coronary artery disease, and hypertension), cerebrovascular disease and peripheral vessel disease; and for the treatment of lupus, polycystic ovary syndrome, carcinogenesis, and hyperplasia. The compounds of the present invention may also be useful for treating physiological disorders related to, for example, cell differentiation to produce lipid accumulating cells, regulation of insulin sensitivity and blood glucose levels, which are involved in, for example, abnormal pancreatic beta cell function, insulin secreting tumors and/or autoimmune hypoglycemia due to autoantibodies to insulin, autoantibodies to the insulin receptor, or autoantibodies that are stimulatory to pancreatic beta cells), macrophage differentiation which leads to the formation of atherosclerotic plaques, inflammatory response, carcinogenesis, hyperplasia, adipocyte gene expression, adipocyte differentiation, reduction in the pancreatic β-cell mass, insulin secretion, tissue sensitivity to insulin, liposarcoma cell growth, polycystic ovarian disease, chronic anovulation, hyperandrogenism, progesterone production, steroidogenesis, redox potential and oxidative stress in cells, nitric oxide synthase (NOS) production, increased gamma glutamyl transpeptidase, catalase, plasma triglycerides, HDL, and LDL cholesterol levels, and the like.

Compounds of the invention may also be used to treat secondary causes of diabetes (Expert Committee on Classification of Diabetes Mellitus, Diabetes Care 22 (Supp. 1):S5, 1999). Such secondary causes include glucocorticoid excess, growth hormone excess, pheochromocytoma, and drug-induced diabetes. Drugs that may induce diabetes include, but are not limited to, pyriminil, nicotinic acid, glucocorticoids, phenytoin, thyroid hormone, β-adrenergic agents, α-interferon and drugs used to treat HIV infection.

Compounds of the present invention may be used alone or in combination with additional therapies and/or compounds known to those skilled in the art in the treatment of diabetes and related disorders. Alternatively, compounds described herein may be used, partially or completely, in combination therapy.

Compounds of the invention may also be administered in combination with other known therapies for the treatment of diabetes, including PPAR agonists, sulfonylurea drugs, non-sulfonylurea secretagogues, α-glucosidase inhibitors, insulin sensitizers, insulin secretagogues, hepatic glucose output lowering compounds, insulin and anti-obesity drugs. Such therapies may be administered prior to, concurrently with or following administration of the compound of the invention. Insulin includes both long and short acting forms and formulations of insulin. PPAR agonist may include agonists of any of the PPAR subunits or combinations thereof. For example, PPAR agonist may include agonists of PPAR-α, PPAR-γ, PPAR-δ or any combination of two or three of the subunits of PPAR. PPAR agonists include, for example, rosiglitazone and pioglitazone. Sulfonylurea drugs include, for example, glyburide, glimepiride, chlorpropamide, and glipizide. α-glucosidase inhibitors that may be useful in treating diabetes when administered with a compound of the invention include acarbose, miglitol and voglibose. Insulin sensitizers that may be useful in treating diabetes when administered with a compound of Formula (I) include thiazolidinediones and non-thiazolidinediones. Hepatic glucose output lowering compounds that may be useful in treating diabetes when administered with a compound of the invention include metformin, such as Glucophage and Glucophage XR. Insulin secretagogues that may be useful in treating diabetes when administered with a compound of the invention include sulfonylurea and non-sulfonylurea drugs: GLP-1, GIP, PACNPAC receptor agonists, secretin, nateglinide, meglitinide, repaglinide, glibenclamide, glimepiride, chlorpropamide, glipizide. GLP-1 includes derivatives of GLP-1 with longer half-lives than native GLP-1, such as, for example, fatty-acid derivatized GLP-1 and exendin. In one embodiment of the invention, compounds of the invention are used in combination with insulin secretagogues to increase the sensitivity of pancreatic β-cells to the insulin secretagogue.

Compounds of the invention may also be used in combination with anti-obesity drugs. Anti-obesity drugs include β-3 agonists, CB-1 antagonists, appetite suppressants, such as, for example, sibutramine (Meridia), and lipase inhibitors, such as, for example, orlistat (Xenical).

Compounds of the invention may also be used in combination with drugs commonly used to treat lipid disorders in diabetic patients. Such drugs include, but are not limited to, HMG-CoA reductase inhibitors, nicotinic acid, bile acid sequestrants, and fibric acid derivatives. Compounds of the invention may also be used in combination with anti-hypertensive drugs, such as, for example, β-blockers and ACE inhibitors.

Such co-therapies may be administered in any combination of two or more drugs (e.g., a compound of the invention in combination with an insulin sensitizer and an anti-obesity drug). Such co-therapies may be administered in the form of pharmaceutical compositions, as described above.

### Terms

As used herein, various terms are defined below.

When introducing elements of the present invention or the preferred embodiment(s) thereof, the articles "a," "an," "the," and "said" are intended to mean that there are one or more of the elements. The terms "comprising," "including," and "having" are intended to be inclusive and mean that there may be additional elements other than the listed elements.

The term "subject" as used herein includes mammals (e.g., humans and animals).

The term "treatment" includes any process, action, application, therapy, or the like, wherein a subject, including a human being, is provided medical aid with the object of improving the subject's condition, directly or indirectly, or slowing the progression of a condition or disorder in the subject.

The term "combination therapy" or "co-therapy" means the administration of two or more therapeutic agents to treat a diabetic condition and/or disorder. Such administration encompasses co-administration of two or more therapeutic agents in a substantially simultaneous manner, such as in a single capsule having a fixed ratio of active ingredients or in multiple, separate capsules for each inhibitor agent. In addition, such administration encompasses use of each type of therapeutic agent in a sequential manner.

The phrase "therapeutically-effective" means the amount of each agent administered that will achieve the goal of improvement in a diabetic condition or disorder severity, while avoiding or minimizing adverse side effects associated with the given therapeutic treatment.

The term "pharmaceutically acceptable" means that the subject item is appropriate for use in a pharmaceutical product.

The term "prodrug" includes a compound that is a drug precursor that, following administration to a subject and subsequent absorption, is converted to an active species in vivo. Conversion to the active, species *in vivo* is typically via some process, such as metabolic conversion. An example of a prodrug is an acylated form of the active compound.

### EVALUATION OF COMPOUNDS

Demonstration of the activity of the compounds of the present invention may be accomplished through *in vitro, ex vivo,* and *in vivo* assays that are well known in the art. For example, to demonstrate the efficacy of a pharmaceutical agent for the treatment of diabetes and related disorders such as Syndrome X, impaired glucose tolerance, impaired fasting glucose, and hyperinsulinemia, the following assays may be used.

### Preparation of pseudo islets in 96-well plates

Pancreata from four Sprague Dawley rats were divided into small pieces approximately 1 mm² or smaller in size. The tissue was then rinsed three times with Hanks-Hepes buffer (127 mM NaCl, 5.4 mM KCl, 0.34 mM Na₂HPO₄, 4.4 mM KH₂PO4, 20 mM HEPES (4-(2-Hydroxyethyl)piperazine-1-ethanesulfonic acid), 1.2 mM CaCl₂/5 mM glucose), and digested with collagenase (Liberase, 0.25 mg/ml, Roche Diagnostic Corp., Indianapolis, IN) at 37°C in a water bath shaker for 10 minutes.

The digested pancreata tissue was rinsed three times with 50 mL Hanks-Hepes buffer to remove the collagenase. The tissue pellet was then filtered through a 250 µm filter and the filtrate was mixed with 16 mL of 27% Ficoll (Sigma, St. Louis, MO, USA) w/v in Hanks-Hepes buffer. Three layers of Ficoll (23%, 20.5%, and 11%, respectively; 8 mL of each concentration) were then loaded on top of the mixture of islet tissue in 27% Ficoll to form a gradient.

The Ficoll gradient was then centrifuged at 1,600 rpm for 10 minutes at room temperature. The pancreatic islets were concentrated at the interphase between 11% and 20.5%, and between 20.5% and 23% depending on the size of islets. The islets were collected from the two interphases and rinsed twice with Ca⁺⁺-free Hanks-Hepes buffer. The islets were then suspended in 5 mL Ca⁺⁺-free Hanks-Hepes buffer containing 1 mM EDTA and incubated for 8 minutes at room temperature.

Trypsin and DNAse I were added to the islet suspension for a final concentration of 25 µg/mL and 2 µg/mL, respectively. This suspension was incubated with shaking at 30°C for 10 minutes. The trypsin digestion was stopped by adding 40 mL RPMI 1640 (GIBCO Life Technologies, Invitrogen, Carlsbad, CA) with 10% FBS. The trypsin digested islet cells were then filtered through a 63 µm nylon filter (PGC Scientific, Frederick, MD) to remove large cell clusters.

The dispersed islet cells were then washed, counted using hemacytometer under the microscope, and seeded into V-bottom 96-well plates (2,500 cells per well). The dispersed islet cell suspension was then centrifuged at 1,000 rpm for 5 minutes. The Hanks-Hepes buffer was removed and replaced with 200 µL RPMI 1640 medium containing 10% FBS, 1% Penicillin - Streptomycin, and 2 mM L-glutamine. Next, the 96-well plates were centrifuged at 1,000 rpm for 5 minutes to collect the dispersed islet cells concentrated at the V-bottom of the plate forming pseudo islets. These pseudo islets were then cultured overnight in a cell culture incubator at 37°C with 5% CO₂, and then used for assays.

### Pseudo islet incubation with 3T3-L1 cells

Dispersed islet cells (prepared by the method described above) were washed with regular RPMI 1640 medium with 10% FBS, counted using hemacytometer under the microscope, and seeded into V-bottom 96-well plates with 3T3-L1 cells (2,500 islet cells and 1,250 3T3-L1 cells per well). The cell suspension was then centrifuged at 1,000 rpm for 5 minutes to collect the dispersed islet cells concentrated at the V-bottom of the plate forming pseudo islets. These pseudo islets were then co-cultured with the 3T3-L1 cells overnight in a cell culture incubator at 37°C with 5% CO₂, and then used for assays.

### Freezing and thawing of pseudo islets

Dispersed islet cells (prepared by the method described in Example 1) were counted as described above and diluted in regular RPMI 1640 medium with 10% FBS and 10% DMSO to a concentration of 2 x 10⁵ cells per mL. An aliquot (1 mL) was transferred to a cryotube and the cryotube was placed in a rack in the vapor phase in a liquid nitrogen tank prior to freezing in liquid nitrogen.

Cells were thawed and then washed with regular medium and seeded into V-bottom 96-well plates (5,000 cells per well). Next, the 96-well plates were centrifuged at 1,000 rpm for 5 minutes to collect the dispersed islet cells concentrated at the V-bottom of the plate forming pseudo islets. These pseudo islets were then cultured overnight in a cell culture incubator at 37°C with 5% CO₂, and then used for assays.

### Static pseudo islet incubation for insulin release assay

Pseudo islets were prepared by the method described above. Following an overnight incubation, the RPMI 1640 medium was removed and replaced by 100 µL Krebs-Ringer-Hepes buffer (115 mM NaCl, 5.0 mM KCl, 24 mM NaHCO₃, 2.2 mM CaCl₂, 1 mM MgCl₂, 20 mM HEPES, 0.25 % BSA (Bovine serum albumin), 0.002% Phenol Red, pH 7.35-7.40). The cell suspension was then centrifuged for 5 minutes at 1,000 rpm to pellet the dispersed islet cells.

Pseudo islets in 96-well plates were incubated in a water bath at 37°C continuously gassed with 95%O₂/5%CO₂ for pre-incubation for 30 minutes. The pre-incubation buffer was removed and replaced with 50 µL incubation buffer (Krebs-Ringer-Hepes buffer, pH 7.35-7.40) containing various test substrates.

The 96-well plate was centrifuged again at 1,000 rpm for 5 minutes to form pseudo islets. These pseudo islets in 96-well plates were statically incubated in a water bath at 37°C continuously gassed with 95%O₂/5%CO₂ for 60 minutes. The incubation buffer (25 µL) was collected after the 60-minute incubation and used for an insulin content assay (ELISA assay, ALPCO, NH, USA).

### Static pseudo islet incubation for insulin biosynthesis

Pseudo islets are prepared as described above. After an overnight culture, the pseudo islets are preincubated in KRBH (Krebs-Ringer-Hepes buffer, 135 mM NaCl, 3.6 mM KCl, 10 mM HEPES, 5 mM NaHCO₃, 0.5 mM NaH₂PO₄, 0.5 mM MgCl₂, 1.5 mM CaCl₂, 0.1% BSA) containing 3 mM glucose for 30 minutes at 37°C, and then incubated for 90 minutes at 37°C with test compounds and 2 µM ³H-Leucine (100 µL) (Amersham, Piscataway, NJ). The pseudo islets are then washed 3x with KRBH containing 1 mM leucine (Sigma, St. Louis, MO), lysed in 2 mM acetic acid (100 µL), sonicated for 15 seconds, and neutralized with 10 N NaOH (20 µL). HEPES (50 mM) containing 0.1% Triton X-100 (Calbiochem, San Diego, CA) is added to bring the volume to 1 mL and the samples are spun for 10 minutes at 1750 x g. Protein A Agarose (50 µL per sample) is preincubated with anti-insulin antibody (Linco, St. Charles, MO) (100 µL per sample) for 2 hours and washed twice. The antibody bead mixture (50 µL) was added to 750 µL of sample and incubated overnight at 4°C. The immunoprecipitates are washed 3x with HEPES (50 mM) containing 0.1% Triton X-100. The beads are then counted in a scintillation counter.

### Static pseudo islet incubation for glucagon release

Pseudo islets are prepared as described above. Following an overnight incubation, the RPMI 1640 medium was removed and replaced by 100 µL Krebs-Ringer-Hepes buffer (115 mM NaCl, 5.0 mM KCl, 24 mM NaHCO₃, 2.2 mM CaCl₂, 1 mM MgCl₂, 20 mM HEPES, 0.25 % BSA, 0.002% Phenol Red, pH 7.35-7.40). The cell suspension was then centrifuged for 5 minutes at 1,000 rpm to pellet the dispersed islet cells.

Pseudo islets in 96-well plates were incubated in a water bath at 37°C continuously gassed with 95%O₂/5%CO₂ for pre-incubation for 30 minutes. The pre-incubation buffer was removed and replaced with 50 µl incubation buffer (Krebs-Ringer-Hepes buffer, pH 7.35-7.40) containing various test compounds.

The 96-well plate was centrifuged again at 1,000 rpm for 5 minutes to form pseudo islets. These pseudo islets in 96-well plates were statically incubated in a water bath at 37°C continuously gassed with 95%O₂/5%CO₂ for 60 minutes. The incubation buffer (25 µL) was collected after the 60-minute incubation and used for a glucagon content assay (Glucagon RIA kit; Linco, St. Charles, MO).

### Assay for identifying insulinotropic compounds

Pseudo islets were prepared as described above. The dispersed islet cells were then washed, counted using a hemacytometer, and seeded into V-bottom 96-well plates (2,500 cells per well) with 200 µL RPMI 1640 medium containing 10% FBS, 1% Penicillin-Streptomycin, and 2 mM L-glutamine. Next, the 96-well plates were centrifuged at 1,000 rpm for 5 minutes to collect the dispersed islet cells concentrated at the V-bottom of the plate forming pseudo islets. These pseudo islets were then cultured overnight in a cell culture incubator at 37°C with 5% CO₂.

Following the overnight incubation, the RPMI 1640 medium was removed and replaced by 100 µL Krebs-Ringer-HEPES buffer (115 mM NaCl, 5.0 mM KC1,24 mM NaHCO₃, 2.2 mM CaCl₂, 1 mM MgCl₂, 20 mM HEPES, 0.25 % BSA, 0.002% Phenol Red, pH 7.35-7.40) with 3 mM glucose. The cell suspension was then centrifuged for 5 minutes at 1,000 rpm to pellet the dispersed islet cells.

The pseudo islets in 96-well plates were incubated in a water bath at 37°C continuously gassed with 95%O₂/5%CO₂ for a pre-incubation of 30 minutes. The pre-incubation buffer was removed and replaced with 50 µL incubation buffer (Krebs-Ringer-HEPES buffer, pH 7.35-7.40) containing the test compounds. The 96-well plates were centrifuged again at 1,000 rpm for 5 minutes to form pseudo islets. These pseudo islets were then statically incubated in a water bath at 37°C continuously gassed with 95%O₂/5%CO₂ for 30 minutes. The incubation buffer (25 µL) was collected after the 30-minute incubation and used for an insulin content assay.

Compounds of the present invention can be tested in vitro for their ability to stimulate insulin release over basal levels in the above assays.

The *in vivo* effect of the compounds according to the invention can be demonstrated in the following assays:
Lean rats (Wistar, male, 250 - 300 g) are fasted over-night and divided into two groups: vehicle and compound treatment (8 rats each group). Vehicle or compound is administrated via gavage (1.5 mL/rat). Two hours later, glucose (30%, 2 g/kg body weight) is injected intraperitoneally. Tail blood samples are collected at 0, 15, 30, and 60 minutes after glucose injection to measure blood glucose using Glucometer (Bayer Diagnostics, Mishawaka, IN).

### Pharmaceutical Compositions

Based on the above tests, or other well known assays used to determine the efficacy for treatment of conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered may generally range from about 0.001 mg/kg to about 200 mg/kg, and preferably from about 0.01 mg/kg to about 200 mg/kg body weight per day. A unit dosage may contain from about 0.05 mg to about 1500 mg of active ingredient, and may be administered one or more times per day. The daily dosage for administration by injection, including intravenous, intramuscular, subcutaneous, and parenteral injections, and use of infusion techniques may be from about 0.01 to about 200 mg/kg. The daily rectal dosage regimen may be from 0.01 to 200 mg/kg of total body weight. The transdermal concentration may be that required to maintain a daily dose of from 0.01 to 200 mg/kg.

Of course, the specific initial and continuing dosage regimen for each patient will vary according to the nature and severity of the condition as determined by the attending diagnostician, the activity of the specific compound employed, the age of the patient, the diet of the patient, time of administration, route of administration, rate of excretion of the drug, drug combinations, and the like. The desired mode of treatment and number of doses of a compound of the present invention or a pharmaceutically acceptable salt thereof may be ascertained by those skilled in the art using conventional treatment tests.

The compounds of this invention may be utilized to achieve the desired pharmacological effect by administration to a patient in need thereof in an appropriately formulated pharmaceutical composition. A patient, for the purpose of this invention, is a mammal, including a human, in need of treatment for a particular condition or disease. Therefore, the present invention includes pharmaceutical compositions which are comprised of a pharmaceutically acceptable carrier and a pharmaceutically effective amount of a compound identified by the methods described herein, or a pharmaceutically acceptable salt or ester thereof. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of a compound is that amount which produces a result or exerts an influence on the particular condition being treated. The compounds identified by the methods described herein may be administered with a pharmaceutically-acceptable carrier using any effective conventional dosage unit forms, including, for example, immediate and timed release preparations, orally, parenterally, topically, or the like.

For oral administration, the compounds may be formulated into solid or liquid preparations such as, for example, capsules, pills, tablets, troches, lozenges, melts, powders, solutions, suspensions, or emulsions, and may be prepared according to methods known to the art for the manufacture of pharmaceutical compositions. The solid unit dosage forms may be a capsule which can be of the ordinary hard- or soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose, and cornstarch in combination with binders such as acacia, cornstarch, or gelatin; disintegrating agents intended to assist the break-up and dissolution of the tablet following administration such as potato starch, alginic acid, corn starch, and guar gum; lubricants intended to improve the flow of tablet granulation and to prevent the adhesion of tablet material to the surfaces of the tablet dies and punches, for example, talc, stearic acid, or magnesium, calcium or zinc stearate; dyes; coloring agents; and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and polyethylene alcohols, either with or without the addition of a pharmaceutically acceptable surfactant, suspending agent, or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent, and one or more preservatives. Suitable dispersing or wetting agents and suspending agents are exemplified by those already mentioned above. Additional excipients, for example, those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, and (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil, or coconut oil; or in a mineral oil such as liquid paraffin. The oily suspensions may contain a thickening agent such as, for example, beeswax, hard paraffin, or cetyl alcohol. The suspensions may also contain one or more preservatives, for example, ethyl or *n*-propyl p-hydroxybenzoate; one or more coloring agents; one or more flavoring agents; and one or more sweetening agents such as sucrose or saccharin.

Syrups and elixirs may be formulated with sweetening agents such as, for example, glycerol, propylene glycol, sorbitol, or sucrose. Such formulations may also contain a demulcent, and preservative, flavoring and coloring agents.

The compounds of this invention may also be administered parenterally, that is, subcutaneously, intravenously, intramuscularly, or interperitoneally, as injectable dosages of the compound in a physiologically acceptable diluent with a pharmaceutical carrier which may be a sterile liquid or mixture of liquids such as water, saline, aqueous dextrose and related sugar solutions; an alcohol such as ethanol, isopropanol, or hexadecyl alcohol; glycols such as propylene glycol or polyethylene glycol; glycerol ketals such as 2,2-dimethyl-1,1-dioxolane-4-methanol, ethers such as poly(ethyleneglycol) 400; an oil; a fatty acid; a fatty acid ester or glyceride; or an acetylated fatty acid glyceride with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention may typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulation ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadecaethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, and isotonic sodium chloride solution. In addition, sterile fixed oils are conventionally employed as solvents or suspending media. For this purpose, any bland, fixed oil may be employed including synthetic mono or diglycerides. In addition, fatty acids such as oleic acid may be used in the preparation of injectables.

A composition of the invention may also be administered in the form of suppositories for rectal administration of the drug. These compositions may be prepared by mixing the drug with a suitable non-irritation excipient which is solid at ordinary temperatures but liquid at the rectal temperature and will therefore melt in the rectum to release the drug. Such material are, for example, cocoa butter and polyethylene glycol.

Another formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art (*see,* e.g., U.S. Patent No. 5,023,252, incorporated herein by reference). Such patches may be constructed for continuous, pulsatile, or on demand delivery of pharmaceutical agents.

It may be desirable or necessary to introduce the pharmaceutical composition to the patient via a mechanical delivery device. The construction and use of mechanical delivery devices for the delivery of pharmaceutical agents is well known in the art. For example, direct techniques for administering a drug directly to the brain usually involve placement of a drug delivery catheter into the patient's ventricular system to bypass the blood-brain barrier. One such implantable delivery system, used for the transport of agents to specific anatomical regions of the body, is described in U.S. Patent No. 5,011,472, incorporated herein by reference.

The compositions of the invention may also contain other conventional pharmaceutically acceptable compounding ingredients, generally referred to as carriers or diluents, as necessary or desired. Any of the compositions of this invention may be preserved by the addition of an antioxidant such as ascorbic acid or by other suitable preservatives. Conventional procedures for preparing such compositions in appropriate dosage forms can be utilized.

Commonly used pharmaceutical ingredients which may be used as appropriate to formulate the composition for its intended route of administration include: acidifying agents, for example, but are not limited to, acetic acid, citric acid, fumaric acid, hydrochloric acid, nitric acid; and alkalinizing agents such as, but are not limited to, ammonia solution, ammonium carbonate, diethanolamine, monoethanolamine, potassium hydroxide, sodium borate, sodium carbonate, sodium hydroxide, triethanolamine, trolamine.

Other pharmaceutical ingredients include, for example, but are not limited to, adsorbents (e.g., powdered cellulose and activated charcoal); aerosol propellants (e.g., carbon dioxide, CCl₂F₂, F₂ClC-CClF₂ and CClF₃); air displacement agents (e.g., nitrogen and argon); antifungal preservatives (e.g., benzoic acid, butylparaben, ethylparaben, methylparaben, propylparaben, sodium benzoate); antimicrobial preservatives (e.g., benzalkonium chloride, benzethonium chloride, benzyl alcohol, cetylpyridinium chloride, chlorobutanol, phenol, phenylethyl alcohol, phenylmercuric nitrate and thimerosal); antioxidants (e.g., ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite); binding materials (e.g., block polymers, natural and synthetic rubber, polyacrylates, polyurethanes, silicones and styrene-butadiene copolymers); buffering agents (e.g., potassium metaphosphate, potassium phosphate monobasic, sodium acetate, sodium citrate anhydrous and sodium citrate dihydrate); carrying agents (e.g., acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection); chelating agents (e.g., edetate disodium and edetic acid); colorants (e.g., FD&C Red No. 3, FD&C Red No. 20, FD&C Yellow No. 6, FD&C Blue No. 2, D&C Green No. 5, D&C Orange No. 5, D&C Red No. 8, caramel and ferric oxide red); clarifying agents (e.g., bentonite); emulsifying agents (but are not limited to, acacia, cetomacrogol, cetyl alcohol, glyceryl monostearate, lecithin, sorbitan monooleate, polyethylene 50 stearate); encapsulating agents (e.g., gelatin and cellulose acetate phthalate); flavorants (e.g., anise oil, cinnamon oil, cocoa, menthol, orange oil, peppermint oil and vanillin); humectants (e.g., glycerin, propylene glycol and sorbitol); levigating agents (e.g., mineral oil and glycerin); oils (e.g., arachis oil, mineral oil, olive oil, peanut oil, sesame oil and vegetable oil); ointment bases (e.g., lanolin, hydrophilic ointment, polyethylene glycol ointment, petrolatum, hydrophilic petrolatum, white ointment, yellow ointment, and rose water ointment); penetration enhancers (transdermal delivery) (e.g., monohydroxy or polyhydroxy alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas); plasticizers (e.g., diethyl phthalate and glycerin); solvents (e.g., alcohol, corn oil, cottonseed oil, glycerin, isopropyl alcohol, mineral oil, oleic acid, peanut oil, purified water, water for injection, sterile water for injection and sterile water for irrigation); stiffening agents (e.g., cetyl alcohol, cetyl esters wax, microcrystalline wax, paraffin, stearyl alcohol, white wax and yellow wax); suppository bases (e.g., cocoa butter and polyethylene glycols (mixtures)); surfactants (e.g., benzalkonium chloride, nonoxynol 10, oxtoxynol 9, polysorbate 80, sodium lauryl sulfate and sorbitan monopalmitate); suspending agents (e.g., agar, bentonite, carbomers, carboxymethylcellulose sodium, hydroxyethyl cellulose, hydroxypropyl cellulose, hydroxypropyl methylcellulose, kaolin, methylcellulose, tragacanth and veegum); sweetening e.g., aspartame, dextrose, glycerin, mannitol, propylene glycol, saccharin sodium, sorbitol and sucrose); tablet anti-adherents (e.g., magnesium stearate and talc); tablet binders (e.g., acacia, alginic acid, carboxymethylcellulose sodium, compressible sugar, ethylcellulose, gelatin, liquid glucose, methylcellulose, povidone and pregelatinized starch); tablet and capsule diluents (e.g., dibasic calcium phosphate, kaolin, lactose, mannitol, microcrystalline cellulose, powdered cellulose, precipitated calcium carbonate, sodium carbonate, sodium phosphate, sorbitol and starch); tablet coating agents (e.g., liquid glucose, hydroxyethyl cellulose, hydroxypropyl cellulose, hydroxypropyl methylcellulose, methylcellulose, ethylcellulose, cellulose acetate phthalate and shellac); tablet direct compression excipients (e.g., dibasic calcium phosphate); tablet disintegrants (e.g., alginic acid, carboxymethylcellulose calcium, microcrystalline cellulose, polacrillin potassium, sodium alginate, sodium starch glycollate and starch); tablet glidants (e.g., colloidal silica, corn starch and talc); tablet lubricants (e.g., calcium stearate, magnesium stearate, mineral oil, stearic acid and zinc stearate); tablet/capsule opaquants (e.g., titanium dioxide); tablet polishing agents (e.g., carnuba wax and white wax); thickening agents (e.g., beeswax, cetyl alcohol and paraffin); tonicity agents (e.g., dextrose and sodium chloride); viscosity increasing agents (e.g., alginic acid, bentonite, carbomers, carboxymethylcellulose sodium, methylcellulose, povidone, sodium alginate and tragacanth); and wetting agents (e.g., heptadecaethylene oxycetanol, lecithins, polyethylene sorbitol monooleate, polyoxyethylene sorbitol monooleate, and polyoxyethylene stearate).

The compounds identified by the methods described herein may be administered as the sole pharmaceutical agent or in combination with one or more other pharmaceutical agents where the combination causes no unacceptable adverse effects. For example, the compounds of this invention can be combined with known anti-obesity, or with known antidiabetic or other indication agents, and the like, as well as with admixtures and combinations thereof.

The compounds identified by the methods described herein may also be utilized, in free base form or in compositions, in research and diagnostics, or as analytical reference standards, and the like. Therefore, the present invention includes compositions which are comprised of an inert carrier and an effective amount of a compound identified by the methods described herein, or a salt or ester thereof. An inert carrier is any material which does not interact with the compound to be carried and which lends support, means of conveyance, bulk, traceable material, and the like to the compound to be carried. An effective amount of compound is that amount which produces a result or exerts an influence on the particular procedure being performed.

Formulations suitable for subcutaneous, intravenous, intramuscular, and the like; suitable pharmaceutical carriers; and techniques for formulation and administration may be prepared by any of the methods well known in the art (*see*, *e.g.,* Remington's Pharmaceutical Sciences, Mack Publishing Co., Easton, Pa., 20^{th} edition, 2000)

The following examples are presented to illustrate the invention described herein, but should not be construed as limiting the scope of the invention in any way.

### Capsule Formulation

A capsule formula is prepared from:

| | |
|---|---|
| Compound of this invention | 40 mg |
| Starch | 109 mg |
| Magnesium stearate | 1 mg |

The components are blended, passed through an appropriate mesh sieve, and filled into hard gelatin capsules.

### Tablet Formulation

A tablet is prepared from:

| | |
|---|---|
| Compound of this invention | 25 mg |
| Cellulose, microcrystaline | 200 mg |
| Colloidal silicon dioxide | 10 mg |
| Stearic acid | 5.0 mg |

The ingredients are mixed and compressed to form tablets. Appropriate aqueous and nonaqueous coatings may be applied to increase palatability, improve elegance and stability or delay absorption.

### Sterile IV Solution

A 5 mg/ml solution of the desired compound of this invention is made using sterile, injectable water, and the pH is adjusted if necessary. The solution is diluted for administration to 1-2 mg/ml with sterile 5% dextrose and is administered as an IV infusion over 60 minutes.

### Intramuscular suspension

The following intramuscular suspension is prepared:

| | |
|---|---|
| Compound of this invention | 50 mg/ml |
| Sodium carboxymethylcellulose | 5 mg/ml |
| TWEEN 80 | 4 mg/ml |
| Sodium chloride | 9 mg/ml |
| Benzyl alcohol | 9 mg/ml |

The suspension is administered intramuscularly.

### Hard Shell Capsules

A large number of unit capsules are prepared by filling standard two-piece hard galantine capsules each with 100 mg of powdered active ingredient, 150 mg of lactose, 50 mg of cellulose and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

### Immediate Release Tablets/Capsules

These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

## Claims

1. A compound of Formula (I) 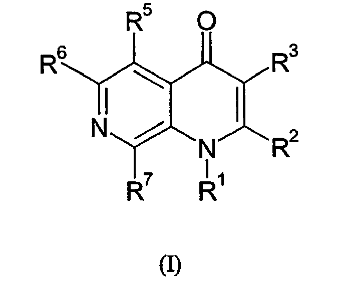 wherein
R¹ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can be substituted with up to 3 substituents selected from the group consisting of nitro, nitrile, halogen, alkyl of 1-4 carbon atoms, and alkoxy of 1-4 carbon atoms,
or
R¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms;
R² is -NR²⁻¹R²⁻² or -SR²⁻³ ;
R²⁻¹ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
or
R²⁻¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻² is selected from the group consisting of hydrogen and alkyl of 1-6 carbon atoms,
or
R²⁻¹ and R²⁻² together with the nitrogen atom to which they are attached, form a heterocycloalkyl of 3-5 carbon atoms and 1-2 heteroatoms selected from N, O, and S, wherein said heterocycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻³ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
or
R²⁻³ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-3 carbon atoms;
R³ is selected from the group consisting of hydrogen, halogen, and alkyl of 1-6 carbon atoms;
R⁵ is selected from the group consisting of hydrogen, nitro, nitrile, halogen, hydroxy, amino, alkyl of 1-6 carbon atoms, haloalkyl of 1-6 carbon atoms, -OR⁵⁻¹, and -NR⁵⁻²R⁵⁻³,
or
R⁵ is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻³ is selected from the group consisting of hydrogen and alkyl of 1-6 carbon atoms;
R⁶ is selected from the group consisting of hydrogen, nitro, nitrile, halogen, hydroxy, amino, alkyl of 1-6 carbon atoms, haloalkyl of 1-6 carbon atoms, -OR⁶⁻¹, and -NR⁶⁻²R⁶⁻³,
or
R⁶ is selected from the group consisting of phenyl, heteroaryl of 3-5 carbon atoms and 1-2 heteroatoms selected from N, O, and S, heterocycloakyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁶⁻³ is selected from the group consisting of hydrogen and alkyl of 1-6 carbon atoms;
R⁷ is selected from the group consisting of hydrogen, nitro, nitrile, halogen, hydroxy, amino, alkyl of 1-6 carbon atoms, haloalkyl of 1-6 carbon atoms, -OR⁷⁻¹, and -NR⁷⁻²R⁷⁻³,
or
R⁷ is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4. carbon atoms, and alkoxy of 1-4 carbon atoms;
R⁷⁻² is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷⁻³ is selected from the group consisting of hydrogen and alkyl of 1-6 carbon atoms;
and a pharmaceutically acceptable salt thereof.

2. A compound according to claim 1,
wherein
R¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms;
R² is -NR²⁻¹R²⁻²;
R²⁻¹ is alkyl of 1-6 carbon atoms, wherein said alkyl can be optionally substituted with phenyl, heteroaryl of 3-5 carbon atoms and 1-2 heteroatoms selected from N, O, and S, or cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms,
or
R²⁻¹ is selected from the group consisting of phenyl, heteroaryl of 3-5 carbon atoms and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻² is selected from the group consisting of hydrogen and alkyl of 1-6 carbon atoms,
or
R²⁻¹ and R²⁻² together with the nitrogen atom to which they are attached, form a heterocycloalkyl, wherein said heterocycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R³ is selected from the group consisting of hydrogen, halogen, and alkyl of 1-6 carbon atoms;
R⁵ is selected from the group consisting of hydrogen, nitro, nitrile, halogen, hydroxy, amino, alkyl of 1-6 carbon atoms, haloalkyl of 1-6 carbon atoms, -OR⁵⁻¹, and -NR⁵⁻²R⁵⁻³,
or
R⁵ is selected from the group consisting of phenyl, heteroaryl of 3-5 carbon atoms and 1-2 heteroatoms selected from N, O, and S, heterocycloalkyl of 3-5 carbon atoms and 1-2 heteroatoms selected from N, O, and S, and cycloalkyl of 3-8 carbon atoms, wherein said phenyl, heteroaryl, heterocycloalkyl, and cycloalkyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, and cycloalkyl of 3-6 carbon atoms;
R⁵⁻² is selected from the group consisting of alkyl of 1-6 carbon atoms, haloalkyl of 1-6 carbon atoms, and cycloalkyl of 3-6 carbon atoms;
R⁵⁻³ is selected from the group consisting of hydrogen and alkyl of 1-6 carbon atoms;
R⁶ is selected from the group consisting of hydrogen, nitro, nitrile, halogen, hydroxy, amino, alkyl of 1-6 carbon atoms, haloalkyl of 1-6 carbon atoms, and -OR⁶⁻¹;
R⁶⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁷ is selected from the group consisting of hydrogen, nitro, nitrile, halogen, hydroxy, amino, alkyl of 1-6 carbon atoms, haloalkyl of 1-6 carbon atoms, and -OR⁷⁻¹;
R⁷⁻¹ is selected from the group consisting of alkyl of 1-6 carbon atoms, haloalkyl of 1-6 carbon atoms, cycloalkyl of 3-6 carbon atoms, phenyl, and heteroaryl of 3-5 carbon atoms and 1-2 heteroatoms selected from N, O, and S, wherein said phenyl and heteroaryl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
and a pharmaceutically acceptable salt thereof.

3. A compound according to claim 1,
wherein
R¹ is selected from the group consisting of phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said phenyl, thienyl, furyl, pyrrolyl, thiazolyl, oxazolyl, imidazolyl, pyridyl, pyrimidyl, pyridazinyl, cyclopropyl, cyclobutyl, cyclopentyl and cyclohexyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms;
R² is -NR²⁻¹R²⁻² ;
R²⁻¹ is selected from the group consisting of phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R²⁻² is hydrogen;
R³ is hydrogen;
R⁵ is selected from the group consisting of hydrogen, nitro, nitrile, halogen, alkyl of 1-6 carbon atoms, haloalkyl of 1-6 carbon atoms, and -OR⁵⁻¹,
or
R⁵ is selected from the group consisting of morpholino, piperazino, piperidino, pyrrolidino, phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl, wherein said morpholino, piperazino, piperidino, pyrrolidino, phenyl, thienyl, furyl, pyrrolyl, thiazolyl, oxazolyl, imidazolyl, pyridyl, pyrimidyl, pyridazinyl, cyclopropyl, cyclobutyl, cyclopentyl, and cyclohexyl can optionally be substituted with up to 3 substituents selected from the group consisting of nitro, nitrile, halogen, hydroxy, alkyl of 1-4 carbon atoms, haloalkyl of 1-4 carbon atoms, and alkoxy of 1-4 carbon atoms;
R⁵⁻¹ is selected from the group consisting of alkyl of 1-6 carbon atoms, haloalkyl of 1-6 carbon atoms, cyclopropyl, cyclobutyl, cyclopentyl, and cyclohexyl;
R⁶ is selected from the group consisting of hydrogen, nitro, nitrile, halogen, alkyl of 1-6 carbon atoms, and haloalkyl of 1-6 carbon atoms;
R⁷ is selected from the group consisting of hydrogen, nitro, nitrile, halogen, hydroxy, amino, alkyl of 1-6 carbon atoms, and haloalkyl of 1-6 carbon atoms;
and a pharmaceutically acceptable salt thereof.

4. A compound according to claim 1,
wherein
R¹ is phenyl, wherein said phenyl can optionally be substituted with up to 1 or 2 substituents selected from the group consisting of nitro, nitrile, fluoro, chloro, methyl, ethyl, propyl, butyl, trifluoromethyl, hydroxy, methoxy, and ethoxy;
R² is -NR²⁻¹R²⁻²;
R²⁻¹ is phenyl wherein said phenyl can optionally be substituted with 1 or 2 substituents selected from the group consisting of nitro, nitrile, fluoro, chloro, hydroxy, methyl, ethyl, propyl, butyl, trifluoromethyl, methoxy, and ethoxy;
R²⁻² is hydrogen;
R³ is hydrogen;
R⁵ is selected from the group consisting of hydrogen, nitro, nitrile, halogen, methyl, ethyl, propyl, butyl, trifluoromethyl, and -OR⁵⁻¹,
or
R⁵ is selected from the group consisting of phenyl, thienyl, pyridyl, pyrimidyl, cyclopropyl, cyclobutyl, cyclopentyl, and cyclohexyl, wherein said phenyl, thienyl, pyridyl, pyrimidyl, cyclopropyl, cyclobutyl, cyclopentyl, and cyclohexyl can optionally be substituted with 1 or 2 substituents selected from the group consisting of nitro, nitrile, fluoro, chloro, hydroxy, methyl, ethyl, propyl, butyl, trifluoromethyl, methoxy and ethoxy;
R⁵⁻¹ is selected from the group consisting methyl, ethyl, propyl, butyl, trifluoromethyl, trifluoroethyl, cyclopropyl, cyclobutyl, cyclopentyl, and cyclohexyl;
R⁶ is selected from hydrogen and methyl;
R⁷ is selected from hydrogen and methyl;
and a pharmaceutically acceptable salt thereof.

5. A compound according to claim 1,
wherein
R¹ is selected from the group consisting of phenyl, heteroaryl of 3-5 carbon atoms, and 1-2 heteroatoms selected from N, O, and S, and cycloalkyl of 3-8 carbon atoms, wherein said phenyl, heteroaryl, and cycloalkyl can optionally be substituted with up to 3 substituents selected from the group consisting of nitro, nitrile, halogen, alkyl of 1-4 carbon atoms, haloalkyl of 1-4 carbon atoms, hydroxy, and alkoxy of 1-4 carbon atoms; and
R² is -NR²⁻¹R²⁻² or -SR²⁻³, wherein R²⁻¹, R²⁻², and R²⁻³ are as defined in claim 1.

6. A pharmaceutical composition comprising an effective amount of a compound of claim 1, or a pharmaceutically acceptable salt thereof, in combination with a pharmaceutically acceptable carrier.

7. A pharmaceutical composition comprising an effective amount of a compound of claim 1, or a pharmaceutically acceptable salt thereof, in combination with a pharmaceutically acceptable carrier and one or more pharmaceutical agents.

8. The pharmaceutical composition of claim 7, wherein said pharmaceutical agent is selected from the group consisting of PPAR agonists, sulfonylurea drugs, non-sulfonylurea secretagogues, α-glucosidase inhibitors, insulin sensitizers, insulin secretagogues, hepatic glucose output lowering compounds, insulin, anti-obesity agents, HMG CoA reductase inhibitors, nicotinic acid, bile acid sequestrants, fibric acid derivatives, and anti-hypertensive agents.

9. A composition comprising an effective amount of a compound of claim 1, or a pharmaceutically acceptable salt thereof, in combination with an inert carrier.

10. Use of a compound of claim 1 for the manufacturing of a medicament for treating diabetes.

11. The use of claim 10, wherein said diabetes is selected from the group consisting of Type 1 diabetes, Type 2 diabetes, maturity-onset diabetes of the young, latent autoimmune diabetes adult, and gestational diabetes.

12. Use of a compound of claim 1 for the manufacturing of a medicament for treating Syndrome X.

13. Use of a compound of claim 1 for the manufacturing of a medicament for treating diabetes-related disorders.

14. The use of claim 13, wherein said diabetes-related disorder is selected from the group consisting of hyperglycemia, hyperinsulinemia, impaired glucose tolerance, impaired fasting glucose, dyslipidemia, hypertriglyceridemia, and insulin resistance.

15. Use of a compound of claim 1 in combination with one or more pharmaceutical agents for the manufacturing of a medicament for treating diabetes.

16. The use of claim 15, wherein said pharmaceutical agent is selected from the group consisting of PPAR agonists, sulfonylurea drugs, non-sulfonylurea secretagogues, α-glucosidase inhibitors, insulin sensitizers, insulin secretagogues, hepatic glucose output lowering compounds, insulin, and anti-obesity agents.

17. The use of claim 16, wherein said diabetes is selected from the group consisting of Type 1 diabetes, Type 2 diabetes, maturity-onset diabetes of the young, latent autoimmune diabetes adult, and gestational diabetes.

18. Use of compound of claim 1 in combination with one or more pharmaceutical agents for the manufacturing of a medicament for treating Syndrome X.

19. The use of claim 18, wherein said pharmaceutical agent is selected from the group consisting of PPAR agonists, sulfonylurea drugs, non-sulfonylurea secretagogues, α-glucosidase inhibitors, insulin sensitizers, insulin secretagogues, hepatic glucose output lowering compounds, insulin, and anti-obesity agents.

20. Use of a compound of claim 1 in combination with one or more pharmaceutical agents for the manufacturing of a medicament for treating diabetes-related disorders.

21. The use of claim 20, wherein said pharmaceutical agent is selected from the group consisting of PPAR agonists, sulfonylurea drugs, non-sulfonylurea secretagogues, α-glucosidase inhibitors, insulin sensitizers, insulin secretagogues, hepatic glucose output lowering compounds, insulin, and anti-obesity agents.

22. The use of claim 21, wherein said diabetes-related disorder is selected from the group consisting of hyperglycemia, hyperinsulinemia, impaired glucose tolerance, impaired fasting glucose, dyslipidemia, hypertriglyceridemia, and insulin resistance.

23. Use of a compound of claim 1 in combination with one or more agents selected from the group consisting of HMG CoA reductase inhibitors, nicotinic acid, bile acid sequestrants, fibric acid derivatives, and anti-hypertensive agents for the manufacturing of a medicament for treating diabetes, Syndrome X, or diabetes-related disorders.

24. The use of claim 23, wherein said diabetes-related disorder is selected from the group consisting of hyperglycemia, hyperinsulinemia, impaired glucose tolerance, impaired fasting glucose, dyslipidemia, hypertriglyceridemia, and insulin resistance.

25. The use of any one of claims 15 to 24, wherein the compound of claim 1 and the one or more pharmaceutical agents are administered as a single pharmaceutical dosage formulation.

26. Use of a compound of claim 1 for the manufacturing of a medicament for treating or preventing secondary causes of diabetes.

27. The use of claim 26, wherein said secondary cause is selected from the group consisting of glucocorticoid excess, growth hormone excess, pheochromocytoma, and drug-induced diabetes.

28. Use of a compound of claim 1 in combination with one or more pharmaceutical agents for the manufacturing of a medicament for treating or preventing secondary causes of diabetes.

29. The use of claim 28, wherein said pharmaceutical agent is selected from the group consisting of PPAR agonists, sulfonylurea drugs, non-sulfonylurea secretagogues, α-glucosidase inhibitors, insulin sensitizers, insulin secretagogues, hepatic glucose output lowering compounds, insulin, and anti-obesity agents.

30. Use of a compound of claim 1 for the manufacturing of a medicament for increasing the sensitivity of pancreatic β-cells to an insulin secretagogue.

31. The use of claim 30, wherein said insulin secretagogue is selected from the group consisting of GLP-1, GIP, PAC/VPAC receptor agonists, secretin, nateglinide, meglitinide, repaglinide, glibenclamide, glimepiride, chlorpropamide, and glipizide.

32. Compounds according to claim 1 for the treatment and/or prophylaxis of disorders.

33. Medicament containing at least one compound according to claim 1 in combination with at least one pharmaceutically acceptable, pharmaceutically safe carrier or excipient.

34. Use of compounds according to claim 1 for manufacturing a medicament for the treatment and/or prophylaxis of diabetes.

35. Medicament according to claim 34 for the treatment and/or prophylaxis of diabetes.

## Patentansprüche

1. Verbindung der Formel (I) 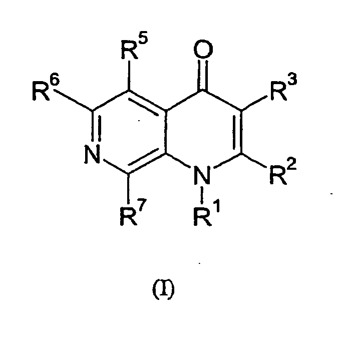 worin
R¹ ein Alkyl mit 1-6 Kohlenstoffatomen ist, worin das Alkyl gegebenenfalls mit Phenyl, Heteroaryl mit 3-5 Kohlenstoffatomen und 1-2 Heteroatomen, ausgewählt aus N, O und S, oder Cycloalkyl mit 3-8 Kohlenstoffatomen substituiert sein kann, worin das Phenyl, Heteroaryl und Cycloalkyl mit bis zu 3 Substituenten, ausgewählt aus der aus Nitro, Nitril, Halogen, Alkyl mit 1-4 Kohlenstoffatomen und Alkoxy mit 1-4 Kohlenstoffatomen bestehenden Gruppe, substituiert sein kann,
oder
R¹ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Alkyl mit 1-4 Kohlenstoffatomen, Halogenalkyl mit 1-4 Kohlenstoffatomen, Hydroxy und Alkoxy mit 1-4 Kohlenstoffatomen bestehenden Gruppe, substituiert sein kann;
R² -NR²⁻¹R²⁻² oder -SR²⁻³ ist;
R²⁻¹ ein Alkyl mit 1-6 Kohlenstoffatomen ist, worin das Alkyl gegebenenfalls mit Phenyl, Heteroaryl mit 3-5 Kohlenstoffatomen und 1-2 Heteroatomen, ausgewählt aus N, O und S, oder Cycloalkyl mit 3-8 Kohlenstoffatomen substituiert sein kann, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann,
oder
R²⁻¹ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R²⁻² aus der aus Wasserstoff und Alkyl mit 1-6 Kohlenstoffatomen bestehenden Gruppe ausgewählt ist,
oder
R²⁻¹ und R²⁻² zusammen mit dem Stickstoffatom, an das sie gebunden sind, ein Heterocycloalkyl mit 3-5 Kohlenstoffatomen und 1-2 Heteroatomen, ausgewählt aus N, O und S, bilden, worin das Heterocycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R²⁻³ ein Alkyl mit 1-6 Kohlenstoffatomen ist, worin das Alkyl gegebenenfalls mit Phenyl, Heteroaryl mit 3-5 Kohlenstoffatomen und 1-2 Heteroatomen, ausgewählt aus N, O und S, oder Cycloalkyl mit 3-8 Kohlenstoffatomen substituiert sein kann, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann,
oder
R²⁻³ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-3 Kohlenstoffatomen bestehenden Gruppe, substituiert sein kann;
R³ aus der aus Wasserstoff, Halogen und Alkyl mit 1-6 Kohlenstoffatomen bestehenden Gruppe ausgewählt ist;
R⁵ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen, -OR⁵⁻¹ und -NR⁵⁻²R⁵⁻³ bestehenden Gruppe ausgewählt ist,
oder
R⁵ aus der aus Phenyl, Heteroaryl mit 3-5 Kohlenstoffatomen und 1-2 Heteroatomen, ausgewählt aus N, O und S, Heterocycloalkyl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl, Heterocycloalkyl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁵⁻¹ aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁵⁻² aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁵⁻³ aus der aus Wasserstoff und Alkyl mit 1-6 Kohlenstoffatomen bestehenden Gruppe ausgewählt ist;
R⁶ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen, -OR⁶⁻¹ und -NR⁶⁻²R⁶⁻³ bestehenden Gruppe ausgewählt ist,
oder
R⁶ aus der aus Phenyl, Heteroaryl mit 3-5 Kohlenstoffatomen und 1-2 Heteroatomen, ausgewählt aus N, O und S, Heterocycloalkyl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl, Heterocycloalkyl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁶⁻¹ aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁶⁻² aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁶⁻³ aus der aus Wasserstoff und Alkyl mit 1-6 Kohlenstoffatomen bestehenden Gruppe ausgewählt ist;
R⁷ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen, -OR⁷⁻¹ und -NR⁷⁻²R⁷⁻³ bestehenden Gruppe ausgewählt ist,
oder
R⁷ aus der aus Phenyl, Heteroaryl mit 3-5 Kohlenstoffatomen und 1-2 Heteroatomen, ausgewählt aus N, O und S, Heterocycloalkyl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl, Heterocycloalkyl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁷⁻¹ aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁷⁻² aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁷⁻³ aus der aus Wasserstoff und Alkyl mit 1-6 Kohlenstoffatomen bestehenden Gruppe ausgewählt ist;
und ein pharmazeutisch annehmbares Salz davon.

2. Verbindung nach Anspruch 1,
worin
R¹ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Alkyl mit 1-4 Kohlenstoffatomen, Halogenalkyl mit 1-4 Kohlenstoffatomen, Hydroxy und Alkoxy mit 1-4 Kohlenstoffatomen bestehenden Gruppe, substituiert sein kann;
R² -NR²⁻¹R²⁻² ist;
R²⁻¹ ein Alkyl mit 1-6 Kohlenstoffatomen ist, worin das Alkyl gegebenenfalls mit Phenyl, Heteroaryl mit 3-5 Kohlenstoffatomen und 1-2 Heteroatomen, ausgewählt aus N, O und S, oder Cycloalkyl mit 3-8 Kohlenstoffatomen substituiert sein kann, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann,
oder
R²⁻¹ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R²⁻² aus der aus Wasserstoff und Alkyl mit 1-6 Kohlenstoffatomen bestehenden Gruppe ausgewählt ist,
oder
R²⁻¹ und R²⁻² zusammen mit dem Stickstoffatom, an das sie gebunden sind, ein Heterocycloalkyl bilden, worin das Heterocycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R³ aus der aus Wasserstoff, Halogen und Alkyl mit 1-6 Kohlenstoffatomen bestehenden Gruppe ausgewählt ist;
R⁵ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen, -OR⁵⁻¹ und -NR⁵⁻²R⁵⁻³ bestehenden Gruppe ausgewählt ist,
oder
R⁵ aus der aus Phenyl, Heteroaryl mit 3-5 Kohlenstoffatomen und 1-2 Heteroatomen, ausgewählt aus N, O und S, Heterocycloalkyl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl, Heterocycloalkyl und Cycloalkyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁵⁻¹ aus der aus Alkyl mit 1-6 Kohlenstoffatomen, Halogenalkyl mit 1-6 Kohlenstoffatomen und Cycloalkyl mit 3-6 Kohlenstoffatomen bestehenden Gruppe ausgewählt ist;
R⁵⁻² aus der aus Alkyl mit 1-6 Kohlenstoffatomen, Halogenalkyl mit 1-6 Kohlenstoffatomen und Cycloalkyl mit 3-6 Kohlenstoffatomen bestehenden Gruppe ausgewählt ist;
R⁵⁻³ aus der aus Wasserstoff und Alkyl mit 1-6 Kohlenstoffatomen bestehenden Gruppe ausgewählt ist;
R⁶ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen und -OR⁶⁻¹ bestehenden Gruppe ausgewählt ist;
R⁶⁻¹ aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁷ aus der aus Wasserstoff, Nitro, Nitril, Halogen, Hydroxy, Amino, Alkyl mit 1-6 Kohlenstoffatomen, Halogenalkyl mit 1-6 Kohlenstoffatomen und -OR⁷⁻¹ bestehenden Gruppe ausgewählt ist,
R⁷⁻¹ aus der aus Alkyl mit 1-6 Kohlenstoffatomen, Halogenalkyl mit 1-6 Kohlenstoffatomen, Cycloalkyl mit 3-6 Kohlenstoffatomen, Phenyl und Heteroaryl mit 3-5 Kohlenstoffatomen und 1-2 Heteroatomen, ausgewählt aus N, O und S, bestehenden Gruppe ausgewählt ist, worin das Phenyl und Heteroaryl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
und ein pharmazeutisch annehmbares Salz davon.

3. Verbindung nach Anspruch 1,
worin
R¹ aus der aus Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl bestehenden Gruppe ausgewählt ist, worin das Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl gegebenenfalls mit bis zu 3 Substituenten, ausgewählt aus der aus Nitro, Nitril, Halogen, Alkyl mit 1-4 Kohlenstoffatomen, Halogenalkyl mit 1-4 Kohlenstoffatomen, Hydroxy und Alkoxy mit 1-4 Kohlenstoffatomen bestehenden Gruppe, substituiert sein kann;
R² -NR²⁻¹R²⁻² ist;
R²⁻¹ aus der aus Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl bestehenden Gruppe ausgewählt ist, worin das Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R²⁻² Wasserstoff ist;
R³ Wasserstoff ist;
R⁵ aus der aus Wasserstoff, Nitro, Nitril, Halogen, Alkyl mit 1-6 Kohlenstoffatomen, Halogenalkyl mit 1-6 Kohlenstoffatomen und -OR⁵⁻¹ bestehenden Gruppe ausgewählt ist,
oder
R⁵ aus der aus Morpholino, Piperazino, Piperidino, Pyrrolidino, Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl bestehenden Gruppe ausgewählt ist, worin das Morpholino, Piperazino, Piperidino, Pyrrolidino, Phenyl, Thienyl, Furyl, Pyrrolyl, Thiazolyl, Oxazolyl, Imidazolyl, Pyridyl, Pyrimidyl, Pyridazinyl, Cyclopropyl, Cyclobutyl, Cyclopentyl und Cyclohexyl gegebenenfalls mit bis zu 3 Substituenten, ausgewählt aus der aus Nitro, Nitril, Halogen, Hydroxy, Alkyl mit 1-4 Kohlenstoffatomen, Halogenalkyl mit 1-4 Kohlenstoffatomen und Alkoxy mit 1-4 Kohlenstoffatomen bestehenden Gruppe, substituiert sein kann;
R⁵⁻¹ aus der aus Alkyl mit 1-6 Kohlenstoffatomen, Halogenalkyl mit 1-6 Kohlenstoffatomen, Cyclopropyl, Cyclobutyl, Cyclopentyl und Cyclohexyl bestehenden Gruppe ausgewählt ist;
R⁶ aus der aus Wasserstoff, Nitro, Nitril, Halogen, Alkyl mit 1-6 Kohlenstoffatomen und Halogenalkyl mit 1-6 Kohlenstoffatomen bestehenden Gruppe ausgewählt ist;
R⁷ aus der aus Wasserstoff, Nitro, Nitril, Halogen, Hydroxy, Amino, Alkyl mit 1-6 Kohlenstoffatomen und Halogenalkyl mit 1-6 Kohlenstoffatomen bestehenden Gruppe ausgewählt ist;
und ein pharmazeutisch annehmbares Salz davon.

4. Verbindung nach Anspruch 1, worin
R¹ Phenyl ist, worin das Phenyl gegebenenfalls mit bis zu 1 oder 2 Substituenten, ausgewählt aus der aus Nitro, Nitril, Fluor, Chlor, Methyl, Ethyl, Propyl, Butyl, Trifluormethyl, Hydroxy, Methoxy und Ethoxy bestehenden Gruppe, substituiert ist;
R² -NR²⁻¹R²⁻² ist;
R²⁻¹ Phenyl ist, worin das Phenyl gegebenenfalls mit bis zu 1 oder 2 Substituenten, ausgewählt aus der aus Nitro, Nitril, Fluor, Chlor, Hydroxy, Methyl, Ethyl, Propyl, Butyl, Trifluormethyl, Methoxy und Ethoxy bestehenden Gruppe, substituiert ist;
R²⁻² Wasserstoff ist;
R³ Wasserstoff ist;
R⁵ aus der aus Wasserstoff, Nitro, Nitril, Halogen, Methyl, Ethyl, Propyl, Butyl, Trifluormethyl und -OR⁵⁻¹ bestehenden Gruppe ausgewählt ist,
oder
R⁵ aus der aus Phenyl, Thienyl, Pyridyl, Pyrimidyl, Cyclopropyl, Cyclobutyl, Cyclopentyl und Cyclohexyl bestehenden Gruppe ausgewählt ist, worin das Phenyl, Thienyl, Pyridyl, Pyrimidyl, Cyclopropyl, Cyclobutyl, Cyclopentyl und Cyclohexyl gegebenenfalls mit 1 bis 2 Substituenten, ausgewählt aus der aus Nitro, Nitril, Fluor, Chlor, Hydroxy, Methyl, Ethyl, Propyl, Butyl, Trifluormethyl, Methoxy und Ethoxy bestehenden Gruppe, substituiert sein kann;
R⁵⁻¹ aus der aus Methyl, Ethyl, Propyl, Butyl, Trifluormethyl, Trifluorethyl, Cyclopropyl, Cyclobutyl, Cyclopentyl und Cyclohexyl bestehenden Gruppe ausgewählt ist;
R⁶ aus Wasserstoff und Methyl ausgewählt ist;
R⁷ aus Wasserstoff und Methyl ausgewählt ist;
und ein pharmazeutisch annehmbares Salz davon.

5. Verbindung nach Anspruch 1,
worin
R¹ aus der aus Phenyl, Heteroaryl mit 3-5 Kohlenstoffatomen und 1-2 Heteroatomen, ausgewählt aus N, O und S, und Cycloalkyl mit 3-8 Kohlenstoffatomen bestehenden Gruppe ausgewählt ist, worin das Phenyl, Heteroaryl und Cycloalkyl gegebenenfalls mit bis zu 3 Substituenten, ausgewählt aus der aus Nitro, Nitril, Halogen, Alkyl mit 1-4 Kohlenstoffatomen, Halogenalkyl mit 1-4 Kohlenstoffatomen, Hydroxy und Alkoxy mit 1-4 Kohlenstoffatomen bestehenden Gruppe, substituiert sein kann; und
R² -NR²⁻¹R²⁻² oder -SR²⁻³ ist, worin R²⁻¹, R²⁻² und R²⁻³ wie in Anspruch 1 definiert sind.

6. Pharmazeutische Zusammensetzung, umfassend eine wirksame Menge einer Verbindung nach Anspruch 1 oder eines pharmazeutisch annehmbaren Salzes davon in Kombination mit einem pharmazeutisch annehmbaren Träger.

7. Pharmazeutische Zusammensetzung, umfassend eine wirksame Menge einer Verbindung nach Anspruch 1 oder eines pharmazeutisch annehmbaren Salzes davon in Kombination mit einem pharmazeutisch annehmbaren Träger und einem oder mehreren pharmazeutischen Mitteln.

8. Pharmazeutische Zusammensetzung nach Anspruch 7, worin das pharmazeutische Mittel aus der aus PPAR-Agonisten, Sulfonylharnstoffwirkstoffen, Nicht-Sulfonylharnstoff-Sekretagoga, α-Glucosidase-Inhibitoren, Insulin-Sensibilisatoren, Insulin-Sekretagoga, die hepatische Glucoseerzeugung reduzierenden Verbindungen, Insulin, Anti-Adipositas-Mitteln, HMG-CoA-Reductase-Inhibitoren, Nicotinsäure, Gallensäure-Maskierungsmittel, Fibratsäurederivaten und blutdrucksenkenden Mitteln bestehenden Gruppe ausgewählt ist.

9. Zusammensetzung, umfassend eine wirksame Menge einer Verbindung nach Anspruch 1 oder eines pharmazeutisch annehmbaren Salzes davon in Kombination mit einem inerten Träger.

10. Verwendung einer Verbindung nach Anspruch 1 zur Herstellung eines Medikaments zur Behandlung von Diabetes.

11. Verwendung nach Anspruch 10, worin der Diabetes aus der aus Typ-1-Diabetes, Typ-2-Diabetes, MODY (sich während der Pubertät manifestierender Diabetes), latentem Autoimmundiabetes bei Erwachsenen und Schwangerschaftsdiabetes bestehenden Gruppe ausgewählt ist.

12. Verwendung einer Verbindung nach Anspruch 1 zur Herstellung eines Medikaments zur Behandlung von Syndrom X.

13. Verwendung einer Verbindung nach Anspruch 1 zur Herstellung eines Medikaments zur Behandlung von mit Diabetes assoziierten Leiden.

14. Verwendung nach Anspruch 13, worin die mit Diabetes assoziierten Leiden aus der aus Hyperglykämie, Hyperinsulinämie, gestörter Glucosetoleranz, abnormer Nüchternglucose, Dyslipidämie, Hypertriglyzeridämie und Insulinresistenz bestehenden Gruppe ausgewählt sind.

15. Verwendung einer Verbindung nach Anspruch 1 in Kombination mit einem oder mehreren pharmazeutischen Mitteln zur Herstellung eines Medikaments zur Behandlung von Diabetes.

16. Verwendung nach Anspruch 15, worin das pharmazeutische Mittel aus der aus PPAR-Agonisten, Sulfonylharnstoffwirkstoffen, Nicht-Sulfonylharnstoff-Sekretagoga, α-Glucosidase-Inhibitoren, Insulin-Sensibilisatoren, Insulin-Sekretagoga, die hepatische Glucoseerzeugung reduzierenden Verbindungen, Insulin und Anti-Adipositas-Mitteln bestehenden Gruppe ausgewählt ist.

17. Verwendung nach Anspruch 16, worin der Diabetes aus der aus Typ-1-Diabetes, Typ-2-Diabetes, MODY, latentem Autoimmundiabetes bei Erwachsenen und Schwangerschaftsdiabetes bestehenden Gruppe ausgewählt ist.

18. Verwendung einer Verbindung nach Anspruch 1 in Kombination mit einem oder mehreren pharmazeutischen Mitteln zur Herstellung eines Medikaments zur Behandlung von Syndrom X.

19. Verwendung nach Anspruch 18, worin das pharmazeutische Mittel aus der aus PPAR-Agonisten, Sulfonylharnstoffwirkstoffen, Nicht-Sulfonylharnstoff-Sekretagoga, α-Glucosidase-Inhibitoren, Insulin-Sensibilisatoren, Insulin-Sekretagoga, die hepatische Glucoseerzeugung reduzierenden Verbindungen, Insulin und Anti-Adipositas-Mitteln bestehenden Gruppe ausgewählt ist.

20. Verwendung einer Verbindung nach Anspruch 1 in Kombination mit einem oder mehreren pharmazeutischen Mitteln zur Herstellung eines Medikaments zur Behandlung von mit Diabetes assoziierten Leiden.

21. Verwendung nach Anspruch 20, worin das pharmazeutische Mittel aus der aus PPAR-Agonisten, Sulfonylharnstoffwirkstoffen, Nicht-Sulfonylharnstoff-Sekretagoga, α-Glucosidase-Inhibitoren, Insulin-Sensibilisatoren, Insulin-Sekretagoga, die hepatische Glucoseerzeugung reduzierenden Verbindungen, Insulin und Anti-Adipositas-Mitteln bestehenden Gruppe ausgewählt ist.

22. Verwendung nach Anspruch 21, worin das mit Diabetes assoziierte Leiden aus der aus Hyperglykämie, Hyperinsulinämie, gestörter Glucosetoleranz, abnormer Nüchternglucose, Dyslipidämie, Hypertriglyzeridämie und Insulinresistenz bestehenden Gruppe ausgewählt ist.

23. Verwendung einer Verbindung nach Anspruch 1 in Kombination mit einem oder mehreren Mitteln, ausgewählt aus der aus HMG-CoA-Reductase-Inhibitoren, Nicotinsäure, Gallensäure-Maskierungsmittel, Fibratsäurederivaten und blutdrucksenkenden Mitteln bestehenden Gruppe, zur Herstellung eines Medikaments zur Behandlung von Diabetes, Syndrom X oder mit Diabetes assoziierten Leiden.

24. Verwendung nach Anspruch 23, worin das mit Diabetes assoziierte Leiden aus der aus Hyperglykämie, Hyperinsulinämie, gestörter Glucosetoleranz, abnormer Nüchternglucose, Dyslipidämie, Hypertriglyzeridämie und Insulinresistenz bestehenden Gruppe ausgewählt ist.

25. Verwendung nach einem der Ansprüche 15 bis 24, worin die Verbindung nach Anspruch 1 und das eine oder die mehreren pharmazeutischen Mittel in Form einer einzelnen pharmazeutischen Dosierungsformulierung verabreicht werden.

26. Verwendung einer Verbindung nach Anspruch 1 zur Herstellung eines Medikaments zur Behandlung oder Prävention von sekundären Ursachen von Diabetes.

27. Verwendung nach Anspruch 26, worin die sekundäre Ursache aus der aus Glucocorticoidüberschuss, Wachstumshormonüberschuss, Phäochromozytom und Wirkstoff-induziertem Diabetes bestehenden Gruppe ausgewählt ist.

28. Verwendung einer Verbindung nach Anspruch 1 in Kombination mit einem oder mehreren pharmazeutischen Mitteln zur Herstellung eines Medikaments zur Behandlung oder Prävention von sekundären Ursachen von Diabetes.

29. Verwendung nach Anspruch 28, worin das pharmazeutische Mittel aus der aus PPAR-Agonisten, Sulfonylharnstoffwirkstoffen, Nicht-Sulfonylharnstoff-Sekretagoga, α-Glucosidase-Inhibitoren, Insulin-Sensibilisatoren, Insulin-Sekretagoga, die hepatische Glucoseerzeugung reduzierenden Verbindungen, Insulin und Anti-Adipositas-Mitteln bestehenden Gruppe ausgewählt ist.

30. Verwendung einer Verbindung nach Anspruch 1 zur Herstellung eines Medikaments zur Steigerung der Empfindlichkeit von Pankreas-β-Zellen auf ein Insulin-Sekretagogum.

31. Verwendung nach Anspruch 30, worin das Insulin-Sekretagogum aus der aus GLP-1, GIP, PAC/VPAC-Rezeptoragonisten, Sekretin, Nateglinid, Meglitinid, Repaglinid, Glibenclamid, Glimepirid, Chlorpropamid und Glipizid bestehenden Gruppe ausgewählt ist.

32. Verbindungen nach Anspruch 1 zur Behandlung und/oder Prophylaxe von Erkrankungen.

33. Medikament, das zumindest eine Verbindung nach Anspruch 1 in Kombination mit zumindest einem pharmazeutisch annehmbaren, pharmazeutisch sicheren Träger oder Exzipienten enthält.

34. Verwendung von Verbindungen nach Anspruch 1 zur Herstellung eines Medikaments zur Behandlung und/oder Prophylaxe von Diabetes.

35. Medikament nach Anspruch 34 zur Behandlung und/oder Prophylaxe von Diabetes.

## Revendications

1. Composé de formule (I) 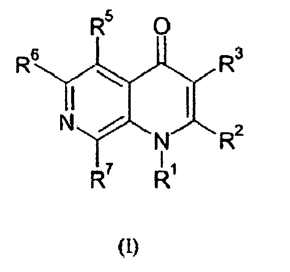 dans laquelle
R¹ représente un groupe alkyle ayant 1 à 6 atomes de carbone, ledit groupe alkyle pouvant être facultativement substitué avec un groupe phényle, hétéroaryle ayant 3 à 5 atomes de carbone, et 1 ou 2 hétéroatomes choisis entre N, O et S, ou cycloalkyle ayant 3 à 8 atomes de carbone, lesdits groupes phényle, hétéroaryle et cycloalkyle pouvant être substitués avec jusqu'à 3 substituants choisis dans le groupe consistant en des substituants nitro, nitrile, halogéno, alkyle ayant 1 à 4 atomes de carbone et alkoxy ayant 1 à 4 atomes de carbone,
ou
R¹ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, alkyle ayant 1 à 4 atomes de carbone, halogénalkyle ayant 1 à 4 atomes de carbone, hydroxy et alkoxy ayant 1 à 4 atomes de carbone,
R² représente un groupe -NR²⁻¹R²⁻² ou -SR²⁻³ ;
R²⁻¹ représente un groupe alkyle ayant 1 à 6 atomes de carbone, ledit groupe alkyle pouvant être facultativement substitué avec un substituant phényle, hétéroaryle ayant 3 à 5 atomes de carbone et 1 ou 2 hétéroatomes choisis entre N, O et S, ou cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone,
ou
R²⁻¹ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R²⁻² est choisi dans le groupe consistant en un atome d'hydrogène et un groupe alkyle ayant 1 à 6 atomes de carbone,
ou
R²⁻¹ et R²⁻², conjointement avec l'atome d'azote auquel ils sont fixés, forment un groupe hétérocycloalkyle ayant 3 à 5 atomes de carbone et 1 ou 2 hétéroatomes choisis entre N, O et S, ledit groupe hétérocycloalkyle pouvant être facultativement substitué avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R²⁻³ représente un groupe alkyle ayant 1 à 6 atomes de carbone, ledit groupe alkyle pouvant être facultativement substitué avec un substituant phényle, hétéroaryle ayant 3 à 5 atomes de carbone et 1 ou 2 hétéroatomes choisis entre N, O et S, ou cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone,
ou
R²⁻³ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 3 atomes de carbone ;
R³ est choisi dans le groupe consistant en un atome d'hydrogène, un atome d'halogène et un groupe alkyle ayant 1 à 6 atomes de carbone ;
R⁵ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone, -OR⁵⁻¹ et -NR⁵⁻² R⁵⁻³ ;
ou
R⁵ est choisi dans le groupe consistant en des groupes phényle, hétéroaryle ayant 3 à 5 atomes de carbone et 1 ou 2 hétéroatomes choisis entre N, O et S, et hétérocycloalkyle ayant 3 à 5 atomes de carbone, et 1 ou 2 hétéroatomes choisis entre N, O et S, et cycloalkyle ayant 3 à 8 atomes de carbone, lesdits groupes phényle, hétéroaryle, hétérocycloalkyle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁵⁻¹ est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁵⁻² est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁵⁻³ est choisi dans le groupe consistant en un atome d'hydrogène et un groupe alkyle ayant 1 à 6 atomes de carbone ;
R⁶ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone, -OR⁶⁻¹ et -NR⁶⁻²R⁶⁻³ ;
ou
R⁶ est choisi dans le groupe consistant en des groupes phényle, hétéroaryle ayant 3 à 5 atomes de carbone et 1 ou 2 hétéroatomes choisis entre N, O et S, hétérocycloalkyle ayant 3 à 5 atomes de carbone, et 1 ou 2 hétéroatomes choisis entre N, O et S, et cycloalkyle ayant 3 à 8 atomes de carbone, lesdits groupes phényle, hétéroaryle, hétérocycloalkyle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁶⁻¹ est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁶⁻² est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁶⁻³ est choisi dans le groupe consistant en un atome d'hydrogène et un groupe alkyle ayant 1 à 6 atomes de carbone ;
R⁷ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone, -OR⁷⁻¹ et -NR⁷⁻²R⁷⁻³ ;
ou
R⁷ est choisi dans le groupe consistant en des groupes phényle, hétéroaryle ayant 3 à 5 atomes de carbone et 1 ou 2 hétéroatomes choisis entre N, O et S, hétérocycloalkyle ayant 3 à 5 atomes de carbone, et 1 ou 2 hétéroatomes choisis entre N, O et S, et cycloalkyle ayant 3 à 8 atomes de carbone, lesdits groupes phényle, hétéroaryle, hétérocycloalkyle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁷⁻¹ est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁷⁻² est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁷⁻³ est choisi dans le groupe consistant en un atome d'hydrogène et un groupe alkyle ayant 1 à 6 atomes de carbone ;
et un de ses sels pharmaceutiquement acceptables.

2. Composé suivant la revendication 1,
dans lequel
R¹ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, alkyle ayant 1 à 4 atomes de carbone, halogénalkyle ayant 1 à 4 atomes de carbone, hydroxy et alkoxy ayant 1 à 4 atomes de carbone,
R² représente un groupe -NR²⁻¹R²⁻² ;
R²⁻¹ représente un groupe alkyle ayant 1 à 6 atomes de carbone, ledit groupe alkyle pouvant être facultativement substitué avec un substituant phényle, hétéroaryle ayant 3 à 5 atomes de carbone et 1 ou 2 hétéroatomes choisis entre N, O et S, ou cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone,
ou
R²⁻¹ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R²⁻² est choisi dans le groupe consistant en un atome d'hydrogène et un groupe alkyle ayant 1 à 6 atomes de carbone,
ou
R²⁻¹ et R²⁻², conjointement avec l'atome d'azote auquel ils sont fixés, forment un groupe hétérocycloalkyle, ledit groupe hétérocycloalkyle pouvant être facultativement substitué avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R³ est choisi dans le groupe consistant en un atome d'hydrogène, un atome d'halogène et un groupe alkyle ayant 1 à 6 atomes de carbone ;
R⁵ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone, -OR⁵⁻¹ et -NR⁵⁻²R⁵⁻³ ;
ou
R⁵ est choisi dans le groupe consistant en des groupes phényle, hétéroaryle ayant 3 à 5 atomes de carbone et 1 ou 2 hétéroatomes choisis entre N, O et S, hétérocycloalkyle ayant 3 à 5 atomes de carbone, et 1 ou 2 hétéroatomes choisis entre N, O et S, et cycloalkyle ayant 3 à 8 atomes de carbone, lesdits groupes phényle, hétéroaryle, hétérocycloalkyle et cycloalkyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁵⁻¹ est choisi dans le groupe consistant en des groupes alkyle ayant 1 à 6 atomes de carbone, halogénalkyle ayant 1 à 6 atomes de carbone et cycloalkyle ayant 3 à 6 atomes de carbone ;
R⁵⁻² est choisi dans le groupe consistant en des groupes alkyle ayant 1 à 6 atomes de carbone, halogénalkyle ayant 1 à 6 atomes de carbone et cycloalkyle ayant 3 à 6 atomes de carbone ;
R⁵⁻³ est choisi dans le groupe consistant en un atome d'hydrogène et un groupe alkyle ayant 1 à 6 atomes de carbone ;
R⁶ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone et -OR⁶⁻¹ ;
R⁶⁻¹ est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁷ est choisi dans le groupe consistant en un atome d'hydrogène, des groupes nitro, nitrile, halogéno, hydroxy, amino, alkyle ayant 1 à 6 atomes de carbone, halogénalkyle ayant 1 à 6 atomes de carbone et -OR⁷⁻¹;
R⁷⁻¹ est choisi dans le groupe consistant en des groupes alkyle ayant 1 à 6 atomes de carbone, halogénalkyle ayant 1 à 6 atomes de carbone, cycloalkyle ayant 3 à 6 atomes de carbone, phényle et hétéroaryle ayant 3 à 5 atomes de carbone et 1 ou 2 hétéroatomes choisis entre N, O et S, lesdits groupes phényle et hétéroaryle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
et un de ses sels pharmaceutiquement acceptables.

3. Composé suivant la revendication 1,
dans lequel
R¹ est choisi dans le groupe consistant en des groupes phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle, lesdits groupes phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle pouvant être facultativement substitués avec jusqu'à 3 substituants choisis dans le groupe consistant en des substituants nitro, nitrile, halogéno, alkyle ayant 1 à 4 atomes de carbone, halogénalkyle ayant 1 à 4 atomes de carbone, hydroxy et alkoxy ayant 1 à 4 atomes de carbone ;
R² représente un groupe -NR²⁻¹R²⁻²
R²⁻¹ est choisi dans le groupe consistant en des groupes phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle, lesdits groupes phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R²⁻² représente un atome d'hydrogène ;
R³ représente un atome d'hydrogène ;
R⁵ est choisi dans le groupe consistant en un atome d'hydrogène, des groupes nitro, nitrile, halogéno, alkyle ayant 1 à 6 atomes de carbone, halogénalkyle ayant 1 à 6 atomes de carbone et -OR⁵⁻¹,
ou
R⁵ est choisi dans le groupe consistant en des groupes morpholino, pipérazino, pipéridino, pyrrolidino, phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle, lesdits groupes morpholino, pipérazino, pipéridino, pyrrolidino, phényle, thiényle, furyle, pyrrolyle, thiazolyle, oxazolyle, imidazolyle, pyridyle, pyrimidyle, pyridazinyle, cyclopropyle, cyclobutyle, cyclopentyle et cyclohexyle pouvant être facultativement substitués avec jusqu'à 3 substituants choisis dans le groupe consistant en des substituants nitro, nitrile, halogéno, hydroxy, alkyle ayant 1 à 4 atomes de carbone, halogénalkyle ayant 1 à 4 atomes de carbone, et alkoxy ayant 1 à 4 atomes de carbone ;
R⁵⁻¹ est choisi dans le groupe consistant en des groupes alkyle ayant 1 à 6 atomes de carbone, halogénalkyle ayant 1 à 6 atomes de carbone, cyclopropyle, cyclobutyle, cyclopentyle et cyclohexyle ;
R⁶ est choisi dans le groupe consistant en un atome d'hydrogène, des groupes nitro, nitrile, halogéno, alkyle ayant 1 à 6 atomes de carbone et halogénalkyle ayant 1 à 6 atomes de carbone ;
R⁷ est choisi dans le groupe consistant en un atome d'hydrogène, des groupes nitro, nitrile, halogéno, hydroxy, amino, alkyle ayant 1 à 6 atomes de carbone et halogénalkyle ayant 1 à 6 atomes de carbone ;
et un de ses sels pharmaceutiquement acceptables.

4. Composé suivant la revendication 1,
dans lequel
R¹ représente un groupe phényle, ledit groupe phényle pouvant être facultativement substitué avec jusqu'à 1 ou 2 substituants choisis dans le groupe consistant en des substituants nitro, nitrile, fluoro, chloro, méthyle, éthyle, propyle, butyle, trifluorométhyle, hydroxy, méthoxy et éthoxy ;
R² représente un groupe -NR²⁻¹R²⁻²
R²⁻¹ représente un groupe phényle, ledit groupe phényle pouvant être facultativement substitué avec jusqu'à 1 ou 2 substituants choisis dans le groupe consistant en des substituants nitro, nitrile, fluoro, chloro, hydroxy, méthyle, éthyle, propyle, butyle, trifluorométhyle, méthoxy et éthoxy ;
R²⁻² représente un atome d'hydrogène ;
R³ représente un atome d'hydrogène ;
R⁵ est choisi dans le groupe consistant en un atome d'hydrogène, des groupes nitro, nitrile, halogéno, méthyle, éthyle, propyle, butyle, trifluorométhyle et -OR⁵⁻¹ ;
ou
R⁵ est choisi dans le groupe consistant en des groupes phényle, thiényle, pyridyle, pyrimidyle, cyclopropyle, cyclobutyle, cyclopentyle et cyclohexyle, lesdits groupes phényle, thiényle, pyridyle, pyrimidyle, cyclopropyle, cyclobutyle, cyclopentyle et cyclohexyle pouvant être facultativement substitués avec jusqu'à 1 ou 2 substituants choisis dans le groupe consistant en des substituants nitro, nitrile, fluoro, chloro, hydroxy, méthyle, éthyle, propyle, butyle, trifluorométhyle, méthoxy et éthoxy ;
R⁵⁻¹ est choisi dans le groupe consistant en des groupes méthyle, éthyle, propyle, butyle, trifluorométhyle, trifluoréthyle, cyclopropyle, cyclobutyle, cyclopentyle et cyclohexyle ;
R⁶ est choisi entre un atome d'hydrogène et un groupe méthyle ;
R⁷ est choisi entre un atome d'hydrogène et un groupe méthyle ;
et un de ses sels pharmaceutiquement acceptables.

5. Composé suivant la revendication 1,
dans lequel
R¹ est choisi dans le groupe consistant en des groupes phényle, hétéroaryle ayant 3 à 5 atomes de carbone et 1 ou 2 hétéroatomes choisis entre N, O et S, et cycloalkyle ayant 3 à 8 atomes de carbone, lesdits groupes phényle, hétéroaryle et cycloalkyle pouvant être facultativement substitués avec jusqu'à 3 substituants choisis dans le groupe consistant en des substituants nitro, nitrile, halogéno, alkyle ayant 1 à 4 atomes de carbone, halogénalkyle ayant 1 à 4 atomes de carbone, hydroxy et alkoxy ayant 1 à 4 atomes de carbone ; et
R² représente un groupe -NR²⁻¹R²⁻² ou -SR²⁻³ , dans lequel R²⁻¹, R²⁻² et R²⁻³ sont tels que définis dans la revendication 1.

6. Composition pharmaceutique comprenant une quantité efficace d'un composé de la revendication 1, ou d'un de ses sels pharmaceutiquement acceptables, en association avec un support pharmaceutiquement acceptable.

7. Composition pharmaceutique comprenant une quantité efficace d'un composé de la revendication 1, ou d'un de ses sels pharmaceutiquement acceptables, en association avec un support pharmaceutiquement acceptable et un ou plusieurs agents pharmaceutiques.

8. Composition pharmaceutique suivant la revendication 7, dans laquelle ledit agent pharmaceutique est choisi dans le groupe consistant en des agonistes de PPAR, des médicaments du type sulfonylurée, des sécrétagogues non du type sulfonylurée, des inhibiteurs d'α-glucosidase, des sensibilisants à l'insuline, des sécrétagogues d'insuline, des composés abaissant l'évacuation du glucose hépatique, l'insuline, des agents anti-obésité, des inhibiteurs de HMG CoA réductase, l'acide nicotinique, des agents séquestrant les acides biliaires, des dérivés d'acide fibrique et des agents antihypertensifs.

9. Composition comprenant une quantité efficace d'un composé de la revendication 1, ou d'un de ses sels pharmaceutiquement acceptables, en association avec un support inerte.

10. Utilisation d'un composé de la revendication 1 pour la production d'un médicament destiné au traitement du diabète.

11. Utilisation suivant la revendication 10, dans laquelle le diabète est choisi dans le groupe consistant en le diabète de type 1, le diabète de type 2, le diabète de la maturité chez le jeune individu, le diabète auto-immun latent de l'adulte et le diabète de la gestation.

12. Utilisation d'un composé de la revendication 1 pour la production d'un médicament destiné au traitement du syndrome X.

13. Utilisation d'un composé de la revendication 1 pour la production d'un médicament destiné au traitement de troubles en rapport avec le diabète.

14. Utilisation suivant la revendication 13, dans laquelle ledit trouble en rapport avec diabète est choisi dans le groupe consistant en l'hyperglycémie, l'hyper-insulinémie, la tolérance entravée au glucose, une perturbation du taux de glucose à jeun, la dyslipidémie, l'hypertriglycéridimie et la résistance à l'insuline.

15. Utilisation d'un composé de la revendication 1 en association avec un ou plusieurs agents pharmaceutiques pour la production d'un médicament destiné au traitement du diabète.

16. Utilisation suivant la revendication 15, dans laquelle ledit agent pharmaceutique est choisi dans le groupe consistant en des agonistes de PPAR, des médicaments du type sulfonylurée, des sécrétagogues non du type sulfonylurée, des inhibiteurs d'α-glucosidase, des sensibilisants à l'insuline, des sécrétagogues d'insuline, des composés abaissant l'évacuation du glucose hépatique, l'insuline et des agents anti-obésité.

17. Utilisation suivant la revendication 16, dans laquelle le diabète est choisi dans le groupe consistant en le diabète de type 1, le diabète de type 2, le diabète de la maturité apparaissant chez le jeune individu, le diabète auto-immun latent de l'adulte et le diabète de la gestation.

18. Utilisation d'un composé de la revendication 1 en association avec un ou plusieurs agents pharmaceutiques pour la production d'un médicament destiné au traitement du syndrome X.

19. Utilisation suivant la revendication 18, dans laquelle ledit agent pharmaceutique est choisi dans le groupe consistant en des agonistes de PPAR, des médicaments du type sulfonylurée, des sécrétagogues non du type sulfonylurée, des inhibiteurs d'α-glucosidase, des sensibilisants à l'insuline, des sécrétagogues d'insuline, des composés abaissant l'évacuation du glucose hépatique, l'insuline et des agents anti-obésité.

20. Utilisation d'un composé de la revendication 1 en association avec un ou plusieurs agents pharmaceutiques pour la production d'un médicament destiné au traitement de troubles en rapport avec le diabète.

21. Utilisation suivant la revendication 20, dans laquelle ledit agent pharmaceutique est choisi dans le groupe consistant en des agonistes de PPAR, des médicaments du type sulfonylurée, des sécrétagogues non du type sulfonylurée, des inhibiteurs d'α-glucosidase, des sensibilisants à l'insuline, des sécrétagogues d'insuline, des composés abaissant l'évacuation du glucose hépatique, l'insuline et des agents anti-obésité.

22. Utilisation suivant la revendication 21, dans laquelle ledit trouble en rapport avec le diabète est choisi dans le groupe consistant en l'hyperglycémie, l'hyper-insulinémie, la tolérance entravée au glucose, la perturbation du taux de glucose à jeun, la dyslipidémie, l'hypertriglycéridémie et la résistance à l'insuline.

23. Utilisation d'un composé de la revendication 1 en association avec un ou plusieurs agents choisis dans le groupe consistant en des inhibiteurs de HMG CoA réductase, de l'acide nicotinique, des agents séquestrant les acides biliaires, des dérivés d'acide fibrique et des agents antihypertensifs pour la production d'un médicament destiné au traitement du diabète, du syndrome X ou de troubles en rapport avec le diabète.

24. Utilisation suivant la revendication 23, dans laquelle ledit trouble en rapport avec le diabète est choisi dans le groupe consistant en l'hyperglycémie, l'hyper-insulinémie, la tolérance entravée au glucose, la perturbation du taux de glucose à jeun, la dyslipidémie, l'hypertriglycéridimie et la résistance à l'insuline.

25. Utilisation suivant l'une quelconque des revendications 15 à 24, dans laquelle le composé de la revendication 1 et le ou les agents pharmaceutiques sont administrés sous forme d'une formulation posologique pharmaceutique unique.

26. Utilisation d'un composé de la revendication 1 pour la production d'un médicament destiné au traitement ou à la prévention des causes secondaires du diabète.

27. Utilisation suivant la revendication 26, dans laquelle ladite cause secondaire est choisie dans le groupe consistant en un excès de glucocorticoïde, un excès d'hormone de croissance, un phéochromocytome et le diabète induit par un médicament.

28. Utilisation d'un composé de la revendication 1 en association avec un ou plusieurs agents pharmaceutiques pour la production d'un médicament destiné au traitement ou à la prévention des causes secondaires du diabète.

29. Utilisation suivant la revendication 28, dans laquelle ledit agent pharmaceutique est choisi dans le groupe consistant en des agonistes de PPAR, des médicaments du type sulfonylurée, des sécrétagogues non du type sulfonylurée, des inhibiteurs d'α-glucosidase, des sensibilisants à l'insuline, des sécrétagogues d'insuline, des composés abaissant l'évacuation du glucose hépatique, l'insuline et des agents anti-obésité.

30. Utilisation d'un composé de la revendication 1 pour la production d'un médicament destiné à augmenter la sensibilité des cellules β-pancréatiques à un sécrétagogue d'insuline.

31. Utilisation suivant la revendication 30, dans laquelle ledit sécrétagogue d'insuline est choisi dans le groupe consistant en GLP-1, GIP, des agonistes des récepteurs de PAC/VPAC, la sécrétine, le natéglinide, le méglitinide, le répaglinide, le glibenclamide, le chlorpropamide et le glipizide.

32. Composés suivant la revendication 1, destinés au traitement et/ou à la prophylaxie d'affections.

33. Médicament contenant au moins un composé suivant la revendication 1, en association avec au moins un support ou excipient pharmaceutiquement acceptable et pharmaceutiquement sûr.

34. Utilisation de composés suivant la revendication 1 pour la production d'un médicament destiné au traitement et/ou à la prophylaxie du diabète.

35. Médicament suivant la revendication 34, destiné au traitement et/ou à la prophylaxie du diabète.
